# EUROPÄISCHE PATENTSCHRIFT

(11) **EP 3 571 263 B1**
(45) Veröffentlichungstag und Bekanntmachung des Hinweises auf die Patenterteilung: **30.03.2022**
(21) Anmeldenummer: 18701313.1
(22) Anmeldetag: 19.01.2018
(51) Int. Cl.: G01N 33/58, C09K 11/08, C09K 11/02, A61P 29/00, A61P 37/06

(54) **VERWENDUNG ZWITTERIONISCHER NANOPARTIKEL**
USE OF ZWITTERIONIC NANOPARTICLES
UTILISATION DE NANOPARTICULES ZWITTERIONIQUES

(30) Priorität: 20.01.2017 DE 102017101057
(43) Veröffentlichungstag der Anmeldung: 27.11.2019
(73) Patentinhaber: Fraunhofer-Gesellschaft zur Förderung der angewandten Forschung e.V., 80686 München (DE); Universitätsklinikum Hamburg-Eppendorf, 20246 Hamburg (DE)
(72) Erfinder: HEEREN, Jörg, 22299 Hamburg (DE); FISCHER, Alexander, 22926 Ahrensburg (DE); SCHOTTEN, Theo, 20146 Hamburg (DE); STEUTER, Michaela, 28201 Bremen (DE); MERKL, Jan-Philip, 82069 Hohenschäftlarn (DE); WELLER, Horst, 22393 Hamburg (DE)
(74) Vertreter: DTS Patent- und Rechtsanwälte Schnekenbühl und Partner mbB
(86) Internationale Anmeldenummer: PCT/EP2018/051288
(87) Internationale Veröffentlichungsnummer: WO 2018/134344

(56) Entgegenhaltungen:
- WO-A2-2013/090601
- US-A1- 2010 258 789
- US-A1- 2010 316 797
- US-A1- 2012 052 513
- ELEONORA MURO ET AL: "Small and Stable Sulfobetaine Zwitterionic Quantum Dots for Functional Live-Cell Imaging", JOURNAL OF THE AMERICAN CHEMICAL SOCIETY, Bd. 132, Nr. 13, 7. April 2010 (2010-04-07), Seiten 4556-4557, XP055000735, ISSN: 0002-7863, DOI: 10.1021/ja1005493

## Beschreibung

### TECHNISCHES FELD

Die vorliegende Erfindung betrifft die Verwendung zwitterionischer Nanopartikel, wobei zwitterionische Verbindungen beschrieben werden, die die Oberfläche von Nanopartikeln bedecken.

### TECHNISCHER HINTERGRUND

Zum technischen Hintergrund gehören z.B. Eleonora Muro et al. Journal of the Amrerican Chemical Society, 2010, Bd. 132, Nr. 13, Seiten 4556-4557; US 2012/052513 A1; US 2010/316797 A1.

Nanopartikel sind von großem Interesse, da sie breite technische Anwendung versprechen, wie in Bildwiedergabegeräten, als Informationsspeicher, als biologische Markierungsmittel, in der diagnostischen Bildgebung, als Wirkstofftransporter, als Theranostika, in der Photovoltaik, als Sensoren und als Katalysatoren. Nanopartikel können infolge ihrer kleinen Abmessungen physikochemische Eigenschaften aufweisen, die zwischen denen von Molekülen und makroskopischen Materialien liegen. Zum Beispiel können Nanopartikel aus Halbleitermaterialien Quanteneffekte, sowohl des Elektrons als und somit auch der Elektronenlücke in allen drei Dimensionen aufweisen, die zu einem Anstieg der Bandlücke des Materials bei sinkender Größe des Kristalliten führt. Auch die magnetischen Eigenschaften der Materie ändern sich dramatisch in Abhängigkeit von der Partikelgröße. Ebenso hängt die Plasmonabsorption von Nanopartikeln, z. B. Goldnanopartikeln überwiegend von der Größe und Form besagter Partikel ab.

Die biologischen Effekte von Nanopartikeln, wie das Durchdringungsvermögen von biologischen Schranken, wie Membranen oder die Haut oder die Toxizität von Nanopartikeln hängen jedoch nicht nur von den Partikeleigenschaften an sich, sondern auch von Veränderungen ab, die durch das Einbringen besagter Partikel in biologische Systeme eintreten. Insbesondere können spontane Auflagerungen biologischer Moleküle stattfinden, die peptidische, protein-, nukleinsäure-, oder kohlenhydrat-artige Strukturelemente aufweisen, wodurch sich eine "biomolekulare Korona" um das ursprüngliche Nanopartikel ausbilden kann, die diesem neuartige, unvorhersehbare und daher meist unerwünschte Eigenschaften verleiht und das weitere biologische Verhalten besagter Partikel maßgeblich bestimmt. Dieser Prozess ist dem Fachmann als Opsonisierung, die sich daraus ergebenden, unerwünschten Eigenschaften u.a. als "Fouling" bekannt. Besagte unerwünschte Effekte stellen ein großes Hindernis bei der Nutzbarmachung von Nanopartikeln für bio-medizinische Anwendungen dar, da für die gezielte Beeinflussung biologischer Vorgänge eine hohe Selektivität bzw. Spezifität besagter Nanopartikel für die biologischen Zielstrukturen, wie Zellen, Gewebe, Organe, Tumoren usw. unabdingbar ist.

Daher wurden zahlreiche Versuche unternommen, besagte unerwünschte Prozesse durch eine entsprechende Oberflächenausstattung besagter Nanopartikel zu unterdrücken und so die damit verbundenen Nachteile zu beseitigen. Insbesondere wurden Poly(ethylenglycol) (PEG) und Oligo(ethylenglycol) (OEG) zur Abdeckung von Nanopartikel-Oberflächen vorgeschlagen. Ebenso wurden zwitterionische Strukturen für diesen Zweck beschrieben, wonach letztere den Stand der Technik abbilden. So beansprucht WO2013090601 (A2) Nanopartikel mit zwitterionischen Liganden, insbesondere um einen geringen Grad von unspezifischer Proteinabsorption zu erreichen. Weiterhin beansprucht WO2013090601 (A2) die Funktionalisierung besagter Nanopartikel mit einer oder mehreren funktionellen Gruppen, um besagten Nanopartikeln die gewünschten Eigenschaften, wie die Bindung an ein Zielmolekül oder an einen Rezeptor zu verleihen.

Somit hat die Belegung von Nanopartikeln mit zwitterionischen Strukturen zum Ziel, die Oberfläche besagter Nanopartikel gegenüber biologischen Einflüssen zu passivieren bzw. zu inertisieren. Dieser Effekt wird in der angelsächsischen Literatur mit "Stealth" bezeichnet. Der gewünschte Effekt ist, dass sich solcher Art inertisierte Nanopartikel zunächst indifferent gegenüber biologischen Einflüssen verhalten, somit für das jeweilige biologische System "unsichtbar" sind und erst durch Anbindung spezifischer Erkennungsmotive für den jeweiligen Einsatzzweck ertüchtigt werden.

Die US 2014/0235803 A1 betrifft nanopartikelbasierte biotechnologische Anwendungen mit gegenüber Opsonisierung unempfindlichen Materialien ("non-fouling materials"), wie Poly(ethylene glycol) (PEG) und oligo(ethylene glycol) (OEG).

Es ist daher die Aufgabe der vorliegenden Erfindung zwitterionische Nanopartikel der eingangs genannten Art in vorteilhafter Weise weiterzubilden, insbesondere dahingehend, dass eine verbesserte und alternative Anwendungsmöglichkeit von Nanopartikeln ermöglicht wird.

### ZUSAMMENFASSUNG DER VORLIEGENDEN ERFINDUNG

Diese Aufgabe wird erfindungsgemäß gelöst durch die Verwendung eines zwitterionischen Nanopartikels mit den Merkmalen des Anspruchs 1. Danach ist vorgesehen, dass ein zwitterionischen Nanopartikels für eine biologische Anwendung verwendet wird, wobei die biologische Anwendung eine immunologische Anwendung ist oder umfasst, wobei das zwitterionische Nanopartikel wenigstens einen Nanopartikel und eine zwitterionische Hülle aufweist, die den Nanopartikel umschließt.

Weitere Ausgestaltungen der vorliegenden Erfindung sind Gegenstand der abhängigen Ansprüche.

Erfindungsgemäß kann die Verwendung in Form einer Zusammensetzung erfolgen, die wenigstens einen zwitterionischen Nanopartikel in wässriger Lösung enthält.

Erfindungsgemäß kann die Verwendung in einer möglichen Ausgestaltung ein Verfahren zur Anbindung eines zwitterionischen Nanopartikels oder einer Zusammensetzung an ein biologisches Molekül oder eine Vielzahl biologischer Moleküle betreffen.

Des Weiteren betrifft die vorliegende Erfindung die Verwendung eines zwitterionischen Nanopartikels für eine in den Patenansprüchen definierte biologische Anwendung.

Überraschend wurde gefunden, dass die vom Stand der Technik beschriebene Vorgehensweise in der vorliegenden Erfindung nicht zu den erwarteten Effekten führte, sondern dass besagte, mit zwitterionischen Strukturen belegte Nanopartikel vielmehr auch ohne Modifikation mit spezifischen Erkennungsmotiven eine überraschend ausgeprägte, strukturabhängige Selektivität bezüglich der Aufnahme in Zellen aufwiesen.

Diese Effekte ließen sich auch *in vivo* nachweisen. So zeigten besagte, mit zwitterionischen Strukturen belegte Nanopartikel eine überraschend hohe Selektivität für die Aufnahme in sinusoidalen Leberendothelzellen (*liver sinusoidal endothelial cells,* LSECs). Aus der Literatur ist bekannt, dass LSECs eine große Rolle in der peripheren Immunregulation durch regulatorische T-Zellen spielen.¹ Nanopartikel, die spezifisch von LSECs aufgenommen werden, können beispielsweise mit bestimmten Autoantigen-Peptiden beladen werden. Durch nanopartikelvermitteltes Targeting können LSECs diese Autoantigen-Peptide prozessieren und präsentieren. Carambia *et al.* zeigten, dass dies zu einer Induktion regulatorischer T-Zellen (Tregs) führt, die das Autoantigen erkennen.² Tregs wirken als Immunmodulatoren. Sie können Immunantworten dämpfen und so zur Toleranz gegenüber Antigenen beitragen. In einem Mausmodell von Multipler Sklerose (MS) wurden Nanopartikel injiziert, an denen Peptide des Myelin-Basischen-Proteins gekoppelt waren. Dies führte zu einer Induktion spezifischer Tregs, die das Fortschreiten der MS unterdrücken konnten.² Dieser Effekt war dabei allein von der Induktion der Tregs durch die LSECs abhängig. Somit könnten QDQRs-17b eine Möglichkeit darstellen, Substanzen spezifisch in LSECs zu transportieren, um immunoregulatorische Effekte zu vermitteln.

Für QDQRs-17b eröffnen sich daher interessante therapeutische Perspektiven, da sie vornehmlich nur in LSECs aber nicht in Kupffer-Zellen aufgenommen werden. Aus der Literatur ist bekannt, dass LSECs eine große Rolle in der peripheren Immunregulation durch regulatorische T-Zellen spielen.¹ Nanopartikel, die spezifisch von LSECs aufgenommen werden, können beispielsweise mit bestimmten Autoantigen-Peptiden gekoppelt werden. Durch nanopartikelvermitteltes Targeting können LSECs diese Autoantigen-Peptide prozessieren und präsentieren. Carambia *et al.* zeigten, dass dies zu einer Induktion regulatorischer T-Zellen (Tregs) führt, die das Autoantigen erkennen.² Tregs wirken als Immunmodulatoren. Sie können Immunantworten dämpfen und so zur Toleranz gegenüber Antigenen beitragen. In einem Mausmodell von Multipler Sklerose (MS) wurden Nanopartikel injiziert, an denen Peptide des Myelin-Basischen-Proteins gekoppelt waren. Dies führte zu einer Induktion spezifischer Tregs, die das Fortschreiten der MS unterdrücken konnten.²Dieser Effekt war dabei allein von der Induktion der Tregs durch die LSECs abhängig. Somit könnten QDQRs-**17b** eine Möglichkeit darstellen, Substanzen spezifisch in LSECs zu transportieren, um immunoregulatorische Effekte zu vermitteln.

Mit anderen Worten:
Erfindungsgemäß ist vorgesehen, dass die biologische Anwendung eine immunologische Anwendung ist oder umfasst. Der Begriff immunologische Anwendung ist dahingehend zu verstehen, dass er eine oder mehrere Anwendung(en) umfasst, die die körperliche Abwehr von Krankheitserregern wie Bakterien, Viren und Pilzen sowie anderen körperfremden Stoffen wie beispielsweise biologischen Toxinen und Umweltgiften betreffen und darüber hinaus von Störungen und Fehlfunktionen dieser Abwehrmechanismen. Immunologische Anwendungen können beispielsweise auf dem Gebiet der Immunchemie, der Immungenetik, der Immunpathologie oder der klinischen Immunologie liegen.

Die immunologische Anwendung kann eine Modulation einer Entzündung sein oder umfassen, insbesondere wobei die Modulation einer Entzündung eine Reduktion autoaggressiver Entzündungen ist oder umfasst.

Des Weiteren kann vorgesehen sein, dass die immunologische Anwendung eine Modulation einer Autoimmunerkrankung ist oder umfasst.

Denkbar ist ein Einsatz im Zusammenhang mit der Behandlung von Multipler Sklerose (MS) oder anderer Autoimmunerkrankungen wie z.B. Lupus erythematodes, Typ 1 Diabetes oder dergleichen.

Außerdem ist denkbar, dass die immunologische Anwendung eine Modulation einer Allergie ist oder umfasst.

Die immunologische Anwendung kann eine Unterdrückung von immunologischen Antworten gegen Biologicals sein oder umfassen. Der Begriff Biologicals wird auch als Biopharmazeutikum bezeichnet. Biopharmazeutika bzw. Biologicals sind Arzneistoffe, die mit Mitteln der Biotechnologie und gentechnisch veränderten Organismen hergestellt werden. Gegen Biologicals, die zur Behandlung von vielen schweren Erkrankungen wie z.B. Tumorerkrankungen und andere eingesetzt werden, können Immunantworten ausgelöst werden, die eine Behandlung der Patienten erschweren bzw. unmöglich machen.

Die biologische Anwendung kann eine Modulation einer Differenzierung einer Zelle, insbesondere einer Leberzelle, beispielsweise einer Leberendothelzelle, sein oder umfassen. Denkbar ist beispielsweise, eine Transdifferenzierung oder Dedifferenzierung einer Zelle, z.B. einer Leberzelle, aufzuhalten oder eine transdifferenzierte Zelle wieder in den Zustand vor der Transdifferenzierung bzw. Dedifferenzierung zurückzuführen bzw. diesen Prozess entsprechen anzustoßen. Denkbar ist ein Einsatz im Zusammenhang mit der Behandlung von Lebererkrankungen wie Leberfibrose, Leberzirrhose, Lebertumoren oder dergleichen.

Somit eignet sich die vorliegende Erfindung hervorragend dazu, durch Strukturvariationen der zwitterionischen Motive auf der Oberfläche von Nanopartikeln, die Aufnahme besagter Nanopartikel in Zellen gezielt zu steuern. Eine solche Anwendung wurde bisher nicht beschrieben und ist somit neu.

### KURZE BESCHREIBUNG DER ABBILDUNGEN

In den Figuren zeigen:
- Fig. 1: eine Quantifizierung der Toxizitätstests der QDQRs-17a und QDQRs-17b sowie Cadmiumchlorid;
- Fig. 2: mikroskopische Aufnahmen der A549-Zellen nach 16-stündiger Inkubationszeit mit unterschiedlichen Konzentrationen von QDQRs-17a;
- Fig. 3: mikroskopische Aufnahmen der A549-Zellen nach 16-stündiger Inkubationszeit mit unterschiedlichen Konzentrationen Cadmiumchlorid;
- Fig. 4: zeigt konfokalmikroskopische Aufnahmen von A549-Zellen nach der Inkubation; und
- Fig. 5: zeigt konfokalmikroskopische Aufnahmen von A549-Zellen nach der Inkubation mit 100 nM QDQRs-17b.
- Fig. 6: zeigt intravitalmikroskopische Aufnahmen der Leber vor und nach der Injektion von QDQRs-17a und QDQRs-17b
- Fig. 7: zeigt intravitalmikroskopische Aufnahmen eines Leberausschnitts nach der Co-Injektion von QDQRs-17a bzw. QDQRs-17b und FluoSpheres^{®}; und
- Fig. 8: zeigt intravitalmikroskopische Aufnahmen eines Leberausschnitts nach der Injektion von QDQRs-17a bzw. QDQRs-17b und histologischer Anfärbung mit einem Endothelmarker
- Fig. 9: zeigt mikroskopische Aufnahmen von QDQRs-**17a und** QDQRs-**17b** in verschiedenen Geweben von Lunge, Milz, Niere und interscapularem braunem Fettgewebe.

### AUSFÜHRLICHE BESCHREIBUNG DER IN DER VORLIEGENDEN ERFINDUNG VERWENDETEN BEGRIFFE

Die vorliegende Erfindung verwendet Nanopartikel mit einer organischen zwitterionischen Hülle, wobei besagte Nanopartikel sich für den Transport von Molekülen, insbesondere Wirkstoffmolekülen eignen und optional superparamagnetische, plasmonische, oder fluoreszente Eigenschaften aufweisen können, einschließlich Kombinationen besagter Eigenschaften. Nanopartikel im Sinne der Erfindung sind räumliche Objekte, die eine Ausdehnung von 1 bis 1000 nm in wenigstens einer Raumrichtung haben. Besagte Nanopartikel können sowohl als feste Partikel, als auch als Hohlkörper in verschiedenen Formen vorliegen, die sphärische, längliche, z. B. stäbchenförmige, elliptische, eiförmige, hantelförmige, ringförmige, würfelförmige oder würfelähnliche, prismatische oder zylindrische, regelmäßig oder unregelmäßig mehrfach facettierte geometrische Körper, vieleckige Platten, von z. B. dreieckiger, quadratischer oder sechseckiger Form, Blättchen, vielarmige oder sternförmige Körper, wie z. B. Tetrapoden, oder drahtartige Gestalt usw. aufweisen. Besagte Nanopartikel können optional mit mindestens einer Schale umhüllt sein, die aus einem anderen Material als der Kern besteht. Die Bezeichnung "Nanopartikel" schließt die Begriffe "Nanokristall" oder "ultrakleines Partikel" mit ein.

Der detaillierten Beschreibung der Erfindung vorangestellt sei, dass diese Erfindung nicht auf die Methoden, Geräte oder Zubereitungen beschränkt ist, da diese selbstverständlich veränderbar sind. Weiterhin sollen die zur Beschreibung der Erfindung verwendeten Begriffe lediglich dazu dienen, bestimmte Ausführungsformen zu erläutern und den Geltungsbereich der Erfindung keinesfalls einschränken. So schließt der bestimmte oder unbestimmte Artikel im Singular "ein" oder "eine" und "der", "die", "das" den Plural ebenfalls mit ein, sofern der Zusammenhang das Gegenteil nicht klar erfordert. Demzufolge schließt z. B. der Bezug "eine Zelle" eine Vielzahl von Zellen, "ein Nanopartikel" eine Vielzahl von Nanopartikeln, "ein Biomolekül", wie beispielsweise DNS, Protein, Peptid, Kohlenhydrat usw. eine Vielzahl von Biomolekülen mit ein, usw.

Alle technischen und wissenschaftlichen Begriffe haben jene Bedeutung, so wie der Fachmann des Sachgebiets der vorliegenden Erfindung sie versteht, sofern nicht anders definiert oder der Zusammenhang eine andere Bedeutung nahelegt. Obwohl auch jede andere Methode oder andere Stoffe, ähnlich oder gleichwertig den hier beschriebenen, geeignet sind die Erfindung auszuüben oder zu erproben, werden im folgenden die bevorzugten Methoden und Stoffe beschrieben.

Alle Publikationen, die hier genannt werden, sind hiermit durch Referenzierung einbezogen für den Zweck der Offenbarung und der Beschreibung der einzelnen Materialien und Verfahrensweisen, für welche die entsprechende Referenz auch zitiert wurde. Diese Publikationen, wie sie hier beschrieben werden, sind lediglich bereitgestellt für deren Offenbarung vor dem Anmeldetag der vorliegenden Erfindung. Hieraus kann jedoch nicht hergeleitet werden, was als Eingeständnis dahingehend gewertet werden kann, dass die Erfindung nicht berechtigt ist, eine derartige Offenbarung als Stand der Technik vorzudatieren. Beim Beschreiben der vorliegenden Erfindung werden die nachstehenden Begriffe benutzt und sollen wie nachfolgend definiert verstanden werden.

Der Begriff "anorganischer Nanokristall" bedeutet ein Partikel, allgemein ein Halbleiter-Partikel, oder ein nanokristalliner Partikel, dem keine Halbleitermaterialien zugrunde liegen, insbesondere ein seltenerd-dotierter Metalloxid-Partikel, oder ein seltenerd-dotierter, salzartiger Partikel, oder ein oxidischer, oder metallischer Partikel mit einem Durchmesser im Bereich von 1 nm bis 1000 nm, vorzugsweise im Bereich von ungefähr 2 nm bis 100 nm. Die Begriffe "Halbleiter Nanokristall" und "Quantum Dot (Quanten-Punkt)", "Quantum Rod, (Quanten-Stäbchen)", "Quantum Dot Rod, (Quantenpunkt/stäbchen)" oder die Abkürzungen QD, QDs, QR, QRs, QDR, QDRs, SCNC oder SCNCs werden hierbei untereinander austauschbar benutzt und bezeichnen Halbleiternanopartikel, die aus einem anorganischen Stoff bestehen, der wahlweise luminiszent ist (d. h. der nach Anregung in der Lage ist, elektromagnetische Strahlung auszusenden), und umfasst einen inneren Kern aus einem oder mehreren ersten Halbleitermaterialien, der wahlweise mit einer Umhüllung oder "Schale" eines zweiten Halbleitermaterials umschlossen ist. Beispielsweise kann das umhüllende Schalenmaterial eine Bandlücken-Energie aufweisen, die höher ist, als jene des Kernmaterials und so ausgewählt ist, dass die Atomabstände ähnlich wie jene des Kernmaterials sind. Ein Halbleiternanokristall der von einer Halbleiterschale umhüllt ist wird als "Kern/Schale-Halbleiternanokristall bezeichnet.

Halbleiter werden insbesondere über ihre elektrische Leitfähigkeit beschrieben, die zwischen der eines Leiters und eines Nicht-Leiters liegt. Geeignete Halbleitermaterialien können folgende Materialien sein, ohne auf diese beschränkt zu sein, und bestehen aus einem ersten Element ausgewählt aus der Gruppe 2 und 12 des Periodensystems der Elemente und einem zweiten Element, ausgewählt aus der Gruppe 16 des Periodensystems (z. B. ZnS, ZnSe, ZnTe, CdS, CdSe, CdTe, HgS, HgSe, HgTe, MgS, MgSe, MgTe, CaS, CaSe, CaTe, SrS, SrSe, SrTe, BaS, BaSe, BaTe, und ähnliche); weiterhin Materialien bestehend aus einem ersten Element, ausgewählt aus der Gruppe 15 des Periodensystems der Elemente und einem zweiten Element aus der Gruppe 15 (z. B. GaN, GaP, GaAs, GaSb, InN, InP, InAs, InSb, usw.); weiterhin Materialien, bestehend aus der Gruppe 14 des Periodensystems der Elemente (Ge, Si usw.); Materialien wie z. B. PbS, PbSe, PbTe usw., Materialien wie CulnS, CulnGaS usw., sowie Legierungen und Mischungen davon. So wie hier verwendet, beziehen sich alle Angaben zum Periodischen System der Elemente und seinen Gruppen auf das neue IUPAC-Nummerierungssystem der Elementgruppen, wie in Handbook of Chemistry and Physics, 61 st 20 Edition (CRC Press, 2000) beschrieben.

Ein SCNC (semi conductor nano crystal) ist wahlweise von einer Schicht eines organischen Materials umschlossen. Diese organische Schicht kann aus einer Vielzahl von Stoffen bestehen und ist dadurch ausgezeichnet, dass sie eine Affinität zu der Oberfläche des SCNC aufweist. Allgemein kann das Beschichtungsmaterial aus kleinen organischen Einzelmolekül, Polymeren (oder Monomeren, die in einer Polymerisationsreaktion eingesetzt werden können), anorganischen Komplexen oder einer ausgedehnten kristallinen Struktur bestehen. Diese Beschichtung kann dazu dienen, die Löslichkeit eines derart beschichteten SCNC in einem gewählten Lösungsmittel, bestimmte Funktionalitäten oder Bindungseigenschaften zu vermitteln. Darüber hinaus kann die Beschichtung auch dazu dienen, die optischen Eigenschaften der SCNC einzustellen. Dementsprechend schließen die Begriffe "Halbleiternanokristall", "SCNC", "Quantum Dot (Quantenpunkt)", wie hier beschrieben, auch organisch ummantelte SCNC-Kerne, sowie organisch ummantelte Kern/Schale-SCNC mit ein.

"Monodisperse Partikel" schließen einen Bestand von Partikeln ein, in dem mindestens 60% vorzugsweise jedoch zwischen 75% und 90% der Partikel des Bestandes, einem gegebenen Größenbereich entsprechen. Ein Bestand monodisperser Partikel hat eine Abweichung des Partikeldurchmessers von weniger als 10%, vorzugsweise jedoch eine Abweichung von weniger als 5% vom quadratischen Mittelwert (root-mean-square, RMS) des Bestandes. Der Ausdruck "eine oder mehrere Größen von SCNC wird gleichbedeutend mit "ein oder mehrere Partikelgrößenverteilungen" verwendet. Der Fachmann wird verstehen, dass bestimmte Größen von Nanopartikeln tatsächlich als Partikelgrößenverteilungen erhalten werden.

Unter "Lumineszenz" wird ein Prozess verstanden, bei dem ein Gegenstand elektromagnetische Strahlung (Licht) aussendet. Lumineszenz entsteht, wenn ein System von einem angeregten Zustand in einen Zustand niedrigerer Energie übergeht, wobei die dabei abgegebene Energie in Form von Photonen freigesetzt wird. Diese Energiezustände können wahlweise in verschiedener Form vorliegen, als Schwingungsenergie oder Rotationsenergie oder elektronischer Natur sein, oder in jeglichen Kombination dieser Energien. Der Übergang, der die Lumineszenz verursacht, kann durch im System gespeicherte chemische Energie oder durch eine äußere Quelle ausgelöst werden. Die äußere Energiequelle kann in verschiedenen Formen, einschließlich chemischer, thermischer, elektrischer, magnetischer, elektromagnetischer, physikalischer, oder jeder anderen Form, die geeignet ist eine Anregung in einen höheren Energiezustand als den Grundzustand zu bewirken, zugeführt werden. Beispielsweise kann ein System durch die Aufnahme wenigstens eines Photons, durch Einbringen in ein elektrisches Feld, oder durch eine chemische Redox-Reaktion angeregt werden. Die Energie des während der Lumineszenz abgestrahlten Photons kann im Bereich niederenergetischer Mikrowellenstrahlung bis hochenergetischer Roentgenstrahlung liegen. Typischerweise bezeichnet Lumineszenz Photonen im Bereich von UV- bis IR-Strahlung.

"Biologisches Molekül" oder "Biomolekül" bezieht sich auf jede Art von Molekülen, die in biologischen Anwendungen vorkommen. Nicht-einschränkend seien aufgezählt: Optional glycosylierte Proteine, z. B. Antikörper, Nanokörper (in der angelsächsischen Literatur: "nano bodies"), Teile von Antikörpern, wie Einzelketten-Antikörper, Fab-Fragmente, virale Proteine, Lektine, Peptide, einschließlich Polyaminosäuren, Nukleinsäuren, einschließlich Desoxyribonukleinsäuren (DNS, DNA), Ribonukleinsäuren (RNS, RNA, siRNA, miRNA) "locked nucleic acid" (LNA), Aptamere, Lipide. Steroide, Botenstoffe, Prione, Kohlenhydrate, kleine Moleküle, usw.

Der Begriff "Mizelle" bezeichnet allgemein eine kolloide Anordnung von Tensiden, die in einer Flüssigkeit verteilt ist und der Begriff "Tensid" bezeichnet eine oberflächenaktive Substanz, die die Oberflächenspannung einer Flüssigkeit herabsetzt, üblicherweise organische Verbindungen, die amphiphil sind. Der Begriff "Transfektion" bezeichnet das beabsichtigte oder unbeabsichtigte Einbringen von Material, so z. B. mizellar verkapselte Nanopartikel in Zellen. Der Begriff "Markieren" oder "Markierung" bezeichnet allgemein das beabsichtigte oder unbeabsichtigte Anheften von Material, so z. B. mizellar verkapselte Nanopartikel an eine Zelle oder ein biologisches Molekül, oder eine biologische Probe. Besagtes Material kann dabei z. B. auf oder in der Zelle angeheftet werden. Transfektion und Markieren einer Zelle oder das Markieren eines biologischen Moleküls, bzw. einer biologischen Probe sind jeweils Beispiele für eine biologische Anwendung. Der Begriff "biologisches Ereignis" schließt eine Wechselwirkung biologischer Strukturen, einen biologischen Prozess, strukturelle Veränderungen in einer biologischen Verbindung oder eine Veränderung in einem biologischen Prozess, ein. Unter dem Begriff "biologische Probe" wird eine Gewebsprobe oder Flüssigkeit verstanden, die aus einem Individuum stammt, einschließlich aber nicht eingeschränkt auf z. B. Plasma, Serum, Spinalflüssigkeit, Sperma, Lymphflüssigkeit, äußere Abschnitte der Haut, des Atmungsapparats, des Verdauungsapparats, des Urogenitaltrakts, Tränenflüssigkeit, Speichel, Milch, Blutzellen, Tumoren, Organe, sowie Proben aus in vitro Zellkultur-Bestandteilen, (einschließlich aber nicht eingeschränkt auf aufbereitetes Zellkulturmedium das aus Zellwachstum in Zellkulturen stammt, wahlweise aus viral infizierten Zellen, rekombinanten Zellen, und Zellbestandteilen) Begriffe wie "biofunktionalisiert", "verbunden", "angeheftet", verknüpft" oder "konjugiert", werden hier gegeneinander austauschbar verwendet und umfassen sowohl direkte als auch indirekte Verknüpfung, Anheftung oder Konjugation, sofern der Zusammenhang nichts anderes verlangt.

Der Begriff "zwitterionisches Nanopartikel" bedeutet hier ein räumliches Objekt, das eine Ausdehnung von 1 bis 1000 nm in wenigstens einer Raumrichtung hat und besagte Ausdehnung von einer zwitterionischen Hülle umschlossen wird. In verschiedenen Ausführungsformen kann diese Hülle aus nicht kovalent mit einander verbundenen Einzelmolekülen bestehen. Die äußere Form der Hülle kann in verschiedenen Ausführungsformen der Erfindung durch ionische, nicht-ionische, elektrostatische, oder kovalente Wechselwirkungen erzeugt werden, auf thermodynamischen, wie z.B. negentropischen Effekten, wie z.B. Mizellenbildung beruhen, oder aus Kombinationen dieser Bindungskräfte. Wahlweise kann die Ausbildung dieser Hülle durch Assoziation der Hüllenkomponenten auf mindestens einem formgebenden anorganischen Nanokristall, wie z.B. einem aus Halbleitern, Silikaten, Salzen, Metallen, oder Metalloxiden bestehender Nanokristall oder auf einem aus organischen Bestandteilen, wie z.B. Polymeren oder festen Lipiden ("solid lipids") bestehenden Nanopartikel oder durch intermolekulare Wechselwirkungen ohne Beteiligung eines formgebenden Nanopartikels erzeugt werden. So stellt die vorliegende Erfindung auch eine Zubereitung bereit, die aus wenigstens einer Mizelle in einer nicht-wässrigen Lösung besteht. Besagte Mizelle kann ein oder mehrere Nanopartikel einkapseln, wobei die Nanopartikel monodisperse Nanopartikel sein können. Besagte Mizelle kann wahlweise anstelle eines oder mehrerer Nanopartikel mindestens einen Wirkstoff enthalten. Besagte Mizelle kann wahlweise leer sein.

Der Begriff "zwitterionische Verbindung" schließt die Begriffe "zwitterionischer Ligand" oder "zwitterionische Gruppe" mit ein. Ebenso sind die Begriffe "Zwitterion" und "Betain" austauschbar.

Besagte zwitterionische Verbindungen haben die allgemeine Formel (I),

A-X-[L¹-Z¹-L²-Z²]ₙ Formel (I)

in der A wahlweise eine Gruppe mit Affinität zur Oberfläche des anorganischen Nanopartikels ist, oder ein mindestens zweiwertiges Atom, vorzugsweise aus der Gruppe C, N, O, S, P oder Si ist, das geeignet ist, eine kovalente Bindung zum Nanopartikel auszubilden. X ist ein optionales Verzweigungselement, dergestalt, dass X zusätzlich zu der Bindung an A mindestens eine weitere Bindung zu L₁ aufweist. X ist wahlweise eine Bindung oder ein mindestens zweiwertiges Atom, vorzugsweise aus der Gruppe C, N, O, S, P oder Si. L¹ und L² sind unabhängig voneinander Linkergruppen; Z¹ schließt eine erste geladene oder ionisierbare Gruppe ein; Z² schließt eine zweite geladene oder ionisierbare Gruppe ein, unter der Maßgabe, dass, falls Z¹ oder Z² Ladungen tragen, diese entgegengesetzt sind. n umfasst die ganzen Zahlen zwischen 1 und der maximalen Valenz von X-1.

A umfasst bzw. weist wahlweise mindestens eine Amino-, Mercapto-, Dithiocarbamat-, Carboxylat-, Phosphat-, Phosphonat-Gruppe auf.

L¹ und L² sind unabhängig voneinander lineare oder verzweigte, wahlweise gesättigte oder ungesättigte Kohlenwasserstoffketten mit ein bis vierzig C-Atomen, die wahlweise Heteroatome aus der Gruppe N, O, P, Si und S enthalten können.

Die folgenden Beispiele dienen zur Veranschaulichung der Erfindung ohne deren Umfang jedoch einzuschränken.

### Referenzbeispiel 1

### Darstellung des tert-Butyl(3-(dimethylamino)propyl)(methyl)carbamats

N,N,N'-Trimethylpropan-1,3-diamin (586 µL, 3,98 mmol) und Triethylamin (TEA) (1,6 mL, 11,54 mmol) wurden in trockenem Dichlormethan (DCM) (12 mL) gelöst und in einem Eisbad abgekühlt. Im Anschluss wurde Di-*tert*-butyldicarbonat (1 mL, 4,35 mmol) in trockenem DCM (2 mL) gelöst und bei einer Temperatur unter 2 °C langsam hinzugetropft. Die Reaktionsmischung wurde für weitere 45 Minuten bei 0 °C und über Nacht (15 Stunden) bei Raumtemperatur gerührt. Zur Aufarbeitung wurde die organische Phase mit gesättigter NaCl, gesättigter NaHCO₃-Lösung und H₂O (je 5 mL) extrahiert, über Na₂SO₄ getrocknet und das Lösungsmittel im Vakuum entfernt. Das Produkt lag in Form eines hellgelben Öls vor.

### Ausbeute: 87%

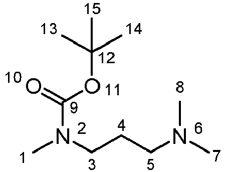

¹H-NMR (600 MHz, CDCl₃): δ [ppm] = 7,26 (CDCl₃); 3,28 - 3,20 (m, 2H, H-3); 2,84 (s, 3H, H-1); 2,31 - 2,25 (m, 2H, H-5); 2,23 (s, 6H, H-7 und H-8); 1,73 - 1,66 (m, 2H, H-4) und 1,45 (s, 9H, H-13, H-14 und H-15).

### Referenzbeispiel 2

### Darstellung des 3-((3-((tert-Butoxycarbonyl)(methyl)amino)propyl)-di-methylammonio)-propan-1-sulfonats

*tert*-Butyl(3-(dimethylamino)propyl)(methyl)carbamat (1,11 g, 5,13 mmol) und 1,3-Propansulton (677 µL, 7,70 mmol) wurden in trockenem DCM (25 mL) gelöst. Die Reaktionsmischung wurde für fünf Tage bei Raumtemperatur gerührt, wobei sich ein perlmuttfarbener Feststoff bildete. Das Lösungsmittel wurde weitestgehend am Rotationsverdampfer entfernt und der Überschuss 1,3-Propansulton durch mehrmaliges Waschen mit trockenem THF abgetrennt. Der Rückstand wurde im Vakuum getrocknet und das Produkt lag als farbloser Feststoff vor.

### Ausbeute: 81%

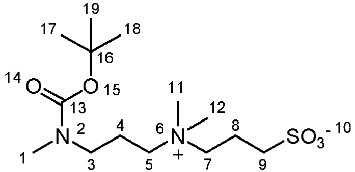

¹H-NMR (400 MHz, D₂O): δ [ppm] = 4,79 (D₂O); 3,55 - 3,51 (m, 2H, H-7); 3,42 - 3,35 (m, 4H, H-3 und H-5); 3,16 (s, 6H, H-11 und H-12); 3,02 (t, 2H, J = 7,2 Hz, H-9), 2,91 (s, 3H, H-1); 2,29-2,21 (m, 2H, H-8), 2,12 - 2,04 (m, 2H, H-4) und 1,50 (s, 9H, H-17, H-18 und H-19).

¹³C-NMR (100 MHz, D₂O): δ [ppm] = 157,6 (C-13); 81,8 (C-16); 62,3 (C-5); 62.0 (C-7); 50,6 (C-11 und C-12); 47,2 (C-9); 45.0 (C-3); 33,8 (C-1); 27,7 (C-17, C-18 und C-19); 20,8 (C-4) und 18,2 (C-8).

ESI-MS: (m/z) ber. für C₁₄H₃₁N₂O₅S (M + H)⁺ 339,1948, gef. 339,1955.

IR (neat): u [cm⁻¹] = 2973; 2925; 1679 (C=O); 1199 (S=O); 1161 (C-O); 1034 (S=O), 605 und 524.

### Referenzbeispiel 3

### Darstellung des 3-(Dimethyl(3-(methylamino)propyl)ammonio)propan-1 -sulfonats

3-((3-((*tert*-Butoxycarbonyl)(methyl)amino)propyl)dimethylammonio)-propan-1-sulfonat (435 mg, 1,29 mmol) wurde in Reinstwasser (13 mL) gelöst und das Reaktionsgefäß mit Stickstoff gespült. Anschließend wurde die Lösung für 90 Minuten in der Mikrowelle auf 135 °C erhitzt. Nach Entfernen des Wassers wurde das Rohprodukt in MeOH (8 mL) gelöst, mit Amberlyst A26 versetzt und vier Stunden bei Raumtemperatur gerührt. Im Anschluss wurde das Amberlyst abfiltriert und das Lösungsmittel am Rotationsverdampfer entfernt. Zur vollständigen Trocknung wurde der Rückstand in wenig Reinstwasser aufgenommen und lyophilisiert. Das Produkt lag als farbloser, stark hygroskopischer Feststoff vor.

### Ausbeute: 88%

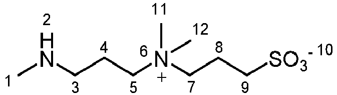

¹H-NMR (400 MHz, D₂O): δ [ppm] = 4,79 (D₂O); 3,52 - 3,48 (m, 2H, H-7); 3,41 - 3,37 (m, 2H, H-5); 3,13 (s, 6 H, H-11 und H-12); 3,00 (t, 2H, J = 7,1 Hz, H-9); 2,76 - 2,72 (m, 2H, H-3); 2,42 (s, 3H, H-1); 2,27-2,19 (m, 2H, H-8) und 2,06 - 1,99 (m, 2H, H-4).

¹³C-NMR (100 MHz, D₂O): δ [ppm] = 62,2 (C-7); 61,9 (C-5); 50,7 (C-11 und C-12); 47,1 (C-9); 46,6 (C-3); 34,1 (C-1); 21,2 (C-4) und 18,1 (C-8).

ESI-MS: (m/z) ber. für C₉H₂₃N₂O₃S (M + H)⁺ 239,1424, gef. 239,1430.

IR (neat): u [cm⁻¹] = 3432; 3034; 2968; 1164 (S=O); 1032 (S=O), 601 und 521.

### Referenzbeispiel 4

### Darstellung des tert-Butyl-bis(3-(dimethylamino)propyl)carbamats

3,3'-Iminobis-N,N-dimethylpropylamin (891 µL, 4,00 mmol) und TEA (1,6 mL, 11,54 mmol) wurden in trockenem DCM (12 mL) gelöst und in einem Eisbad abgekühlt. Im Anschluss wurde Di-*tert*-butyldicarbonat (1 mL, 4,35 mmol) in trockenem DCM (2 mL) gelöst und bei einer Temperatur unter 3 °C langsam hinzugetropft. Die Reaktionsmischung wurde für weitere 90 Minuten bei 1 °C und über Nacht (15 Stunden) bei Raumtemperatur gerührt. Zur Aufarbeitung wurde die organische Phase mit gesättigter NaCl, gesättigter NaHCO₃-Lösung und H₂O (je 5 mL) extrahiert, über Na₂SO₄ getrocknet und das Lösungsmittel im Vakuum entfernt. Das Produkt lag in Form eines hellgelben Öls vor.

### Ausbeute: 68%

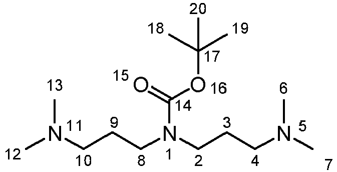

¹H-NMR (400 MHz, CDCl₃): δ [ppm] = 7,26 ppm (CDCl₃); 3,24 - 3,17 (m, 4H, H-2 und H-8); 2,25 (t, 4H, J = 7,4 Hz, H-4 und H-10); 2,21 (s, 12H, H-6, H-7, H-12 und H-13); 1,72 - 1,65 (m, 4H, H-3 und H-9) und 1,45 (s, 9H, H-18, H-19 und H-20).

### Referenzbeispiel 5

### Darstellung des 3,3'-((((tert-Butoxycarbonyl)azadiyl)bis(propan-3,1-diyl))bis(dimethyl-ammoniodiyl))bis(propan-1 -sulfonats)

*tert*-Butyl-bis(3-(dimethylamino)propyl)carbamat (500 mg, 1,74 mmol) und 1,3-Propansulton (459 µL, 5,22 mmol) wurden in trockenem DCM (20 mL) gelöst. Nach 24 Stunden hatte sich ein farbloser Feststoff gebildet und die entstandene Suspension war so dickflüssig, dass erneut trockenes DCM (20 mL) hinzugefügt wurde. Die Reaktionsmischung wurde weitere drei Tage bei Raumtemperatur gerührt. Das Lösungsmittel wurde weitestgehend am Rotationsverdampfer entfernt und der Überschuss 1,3-Propansulton durch mehrmaliges Waschen mit trockenem THF abgetrennt. Der Rückstand wurde im Vakuum getrocknet und das Produkt lag als farbloser Feststoff vor.

### Ausbeute: 91%

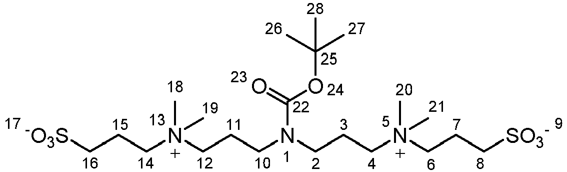

¹H-NMR (400 MHz, D₂O): δ [ppm] = 4,79 (D₂O); 3,55 - 3,51 (m, 4H, H-6 und H-14); 3,40 - 3,36 (m, 8H, H-2, H-4, H-10 und H-12); 3,16 (s, 12H, H-18, H-19, H-20 und H-21); 3,02 (t, 4H, J = 7,1 Hz, H-8 und H-16); 2,27 - 2,21 (m, 4H, H-7 und H-15); 2,13 - 2,05 (m, 4H, H-3 und H-11) und 1,52 (s, 9H, H-26, H-27 und H-28).

¹³C-NMR (100 MHz, D₂O): δ [ppm] = 157,1 (C-22); 82,3 (C-25); 62,2 (C-4, C-6, C-12 und C-14); 50,8 (C-18, C-19, C-20 und C-21); 47,2 (C-8 und C-16); 40,3 (C-2 und C-10); 27,7 (C-26, C-27 und C-28); 22,7 (C-3 und C-11) und 18,2 (C-7 und C-15).

ESI-MS: (m/z) ber. für C₂₁H₄₆N₃O₈S₂ (M + H)⁺ 532,2721, gef. 532,2741.

IR (neat): u [cm⁻¹] = 3452; 3038; 2974; 1676 (C=0), 1157 (C-O); 1033 (S=0), 602 und 521.

### Referenzbeispiel 6

### Darstellung des 3,3'-((Azadiylbis(propan-3,1 -diyl))bis(dimethylammoniodiyl))bis-(propan-1-sulfonats)

3,3'-((((*tert*-Butoxycarbonyl)azanediyl)bis(propan-3,1-diyl))bis(dimethylammoniodiyl))bis-(propan-1-sulfonat) (783 mg, 1,47 mmol) wurde in Reinstwasser (14,7 mL) gelöst. Das Reaktionsgefäß wurde mit Stickstoff gespült und die Lösung wurde für 90 Minuten in der Mikrowelle auf 135 °C erhitzt. Nach Lyophilisation wurde das Produkt in MeOH (10 mL) gelöst und über Nacht in Gegenwart von Amberlyst A 26 gerührt. Nach Abfiltrieren des Amberlyst wurde das Lösungsmittel am Rotationsverdampfer entfernt und der Rückstand nach Zugabe von wenig Reinstwasser erneut lyophilisiert. Das Produkt lag in Form eines hellgelben, schaumartigen Feststoffes vor.

### Ausbeute: 71%

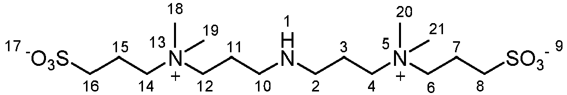

¹H-NMR (400 MHz, D₂O): δ [ppm] = 4,79 (D₂O); 3,55 - 3,51 (m, 4H, H-4 und H-12); 3,44 - 3,39 (4H, m, H-6 und H-14); 3,16 (s, 12 H, H-18, H-19, H-20 und H-21); 3,03 (t, 4H, J = 7,1 Hz, H-8 und H-16); 2,77 - 2,74 (m, 4H, H-2 und H-10); 2,30 - 2,22 (m, 4H, H-7 und H-15) und 2,07 - 1,99 (m, 4H, H-3 und H-11).

¹³C-NMR (100 MHz, D₂O): δ [ppm] = 62,2 (C-4 und C-12); 62,00 (C-6 und C-14); 50,7 (C-18, C-19, C-20 und C-21); 47,2 (C-8 und C-16); 44,9 (C-2 und C-10); 21,7 (C-7 und C-15) und 18,1 (C-3 und C-11).

ESI-MS: (m/z) ber. für C₁₆H₃₈N₃O₆S₂ (M + H)⁺ 432,2197, gef. 432,2216.

IR (neat): u [cm⁻¹] = 3434; 3038; 2968; 2824; 1167 (S=O); 1033 (S=O), 602 und 521.

### Referenzbeispiel 7

### Darstellung des 1-((3-((tert-Butoxycarbonyl)(methyl)amino)dimethyl-ammonio)hex-5-en-3-sulfonats

*tert*-Butyl(3-(dimethylamino)propyl)(methyl)carbamat (434 mg, 2,01 mmol) und 3-Allyl-1,2-oxathiolan-2,2-dioxid (442 mg, 2,72 mmol) wurden in trockenem DCM (20 mL) gelöst. Die Reaktionsmischung wurde für fünf Tage bei Raumtemperatur gerührt, wobei sich ein perlmuttfarbener Feststoff bildete. Das Lösungsmittel wurde weitestgehend am Rotationsverdampfer entfernt und der Überschuss 3-Allyl-1,2-oxathiolan-2,2-dioxid durch mehrmaliges Waschen mit trockenem THF abgetrennt. Der Rückstand wurde im Vakuum getrocknet und das Produkt lag als farbloser Feststoff vor.

### Ausbeute: 83%

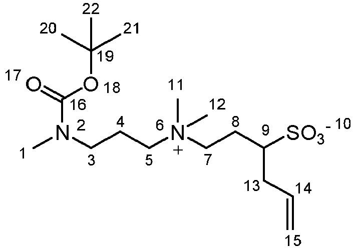

¹H-NMR (400 MHz, D₂O): δ [ppm] = 5,98 - 5,87 (m, 1H, H-14); 5,31 -5,22 (m, 2H, H-15); 4,79 (D₂O); 3,67 - 3,60 (m, 1H, H-7a); 3,55 - 3,31 (m, 5H, H-3, H-5 und H-7b); 3,14 (s, 6H, H-11 und H-12); 3,04 - 2,98 (m, 1H, H-9), 2,91 (s, 3H, H-1); 2,79 - 2,72 (m, 1H, H-13a); 2,42 - 2,36 (m, 1H, H-13b); 2,26 - 2,14 (m, 2H, H-8), 2,08 - 2,03 (m, 2H, H-4) und 1,50 (s, 9H, H-20, H-21 und H-22).

¹³C-NMR (100 MHz, D₂O): δ [ppm] = 157,1 (C-16); 134,2 (C-14); 118,4 (C-15); 81,8 (C-19); 61,6 (C-5 und C-7); 56,5 (C-9); 50,8 (C-11 und C-12); 45,3 (C-3); 34,0 (C-13); 33,7 (C-1); 27,7 (C-20, C-21 und C-22); 22,2 (C-8) und 20,8 (C-4).

ESI-MS: (m/z) ber. für C₁₇H₃₅N₂O₅S (M + H)⁺ 379,2261, gef. 379,2284.

IR (neat): u [cm⁻¹] = 2976; 1679 (C=O); 1199 (S=O); 1172 (C-O); 1028 (S=O), 602 und 537.

### Referenzbeispiel 8

### Darstellung des 1-(Dimethyl(3-methylamino)propyl)ammonio)hex-5-en-3-sulfonats

1-((3-((*tert*-Butoxycarbonyl)(methyl)amino)dimethylammonio)hex-5-en-3-sulfonat (568 mg, 1,50 mmol) wurde in Reinstwasser (15 mL) gelöst. Das Reaktionsgefäß wurde mit Stickstoff gespült und die Lösung wurde für 90 Minuten in der Mikrowelle auf 135 °C erhitzt. Nach Lyophilisation wurde das Produkt in MeOH (10 mL) gelöst und über Nacht in Gegenwart von Amberlyst A 26 gerührt. Nach Abfiltrieren des Amberlyst wurde das Lösungsmittel am Rotationsverdampfer entfernt und der Rückstand nach Zugabe von wenig Reinstwasser erneut lyophilisiert. Das Produkt lag in Form eines hellgelben, schaumartigen Feststoffes vor.

### Ausbeute: 90%

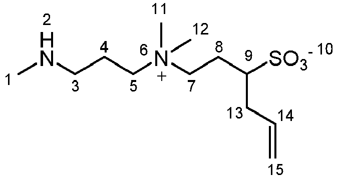

¹H-NMR (400 MHz, D₂O): δ [ppm] = 5,97 - 5,87 (m, 1H, H-14); 5,31 - 5,23 (m, 2H, H-15); 4,79 (D₂O); 3,68 - 3,60 (m, 1H, H-7a); 3,54 - 3,47 (m, 1H, H-7b); 3,41 - 3,37 (m, 2H, H-5); 3,13 (s, 6H, H-11 und H-12); 3,04 - 2,98 (m, 1H, H-9); 2,79 - 2,72 (m, 3H, H-3 und H-13a); 2,43 (s, 3H, H-1); 2,42 - 2,36 (m, 1H, H-13b); 2,27 - 2,11 (m, 2H, H-8) und 2,06 - 1,98 (m, 2H, H-4).

¹³C-NMR (100 MHz, D₂O): δ [ppm] = 134,2 (C-14); 118,4 (C-15); 61, 7 (C-5); 61,5 (C-7); 56,5 (C-9); 50,7 (C-11 und C-12); 46,7 (C-3); 34,2 (C-1); 34,0 (C-13); 22,2 (C-8) und 21,3 (C-4).

ESI-MS: (m/z) ber. für C₁₂H₂₇N₂O₃S (M + H)⁺ 279,1737, gef. 279,1751.

IR (neat): u [cm⁻¹] = 3453; 3028; 2942; 2847; 2793; 1173 (S=O); 1028 (S=O), 601 und 532.

### Referenzbeispiel 9

### Darstellung des 11-Brom-N-methylundecanamids

11 -Bromundecansäure (5,00 g, 18,85 mmol) wurde in trockenem DCM (100 mL) gelöst und innerhalb von fünf Minuten wurde eine Lösung aus Oxalylchlorid (1,7 mL, 19,82 mmol) in trockenem DCM (10 mL) zugetropft. Es wurde zu Beginn eine Gasentwicklung beobachtet und die Reaktionsmischung wurde für eine Stunde bei Raumtemperatur gerührt. Im Anschluss wurde das Lösungsmittel und überschüssiges Oxalylchlorid im Vakuum entfernt und das Rohprodukt in trockenem Et₂O (12 mL) gelöst. Die aktivierte Säure wurde zu einer im Eisbad gekühlten wässrigen Methylaminlösung (40%ig, 4,3 mL, 49,80 mmol) getropft, wobei sich sofort ein farbloser Niederschlag bildete. Der Niederschlag wurde abfiltriert und mit H₂O (50 mL) gewaschen. Das Produkt lag nach Trocknung im Vakuum als farbloser Feststoff vor.

### Ausbeute: 89%

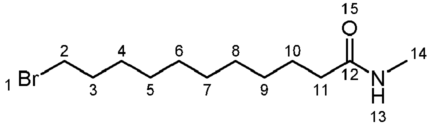

¹H-NMR (400 MHz, CDCl₃): δ [ppm] = 7,26 (CDCl₃); 5,82 (brs, 1H, NH); 3,40 (t, 2H, J = 6,9 Hz, H-2); 2,82 (s, 3H, H-14); 2,21 (t, 2H, J = 7,6 Hz, H-11); 1,88 - 1,81 (m, 2H, H-3); 1,65 - 1,60 (m, 2H, H-10); 1,43 - 1,38 (m, 2H, H-4) und 1,34 - 1,25 (m, 10H, H-5 bis H-9).

### Referenzbeispiel 10

### Darstellung des 11-(Dimethylamino)-N-methylundecanamids

11-Brom-N-methylundecanamid (1,39 g, 5,00 mmol) und eine Lösung aus Dimethylamin in EtOH (33 Gew.%, 9 mL, 50,40 mmol) wurden für zwei Stunden in der Mikrowelle auf 60 °C erhitzt. Das Lösungsmittel und überschüssiges Dimethylamin wurden im Vakuum entfernt und das Hydrobromid des Produkts wurde in Form eines hellgelben Feststoffes erhalten. Der Feststoff wurde in MTBE (10 mL) suspendiert und mit 1 M NaOH (5 mL) extrahiert. Die organische Phase wurde über Na₂SO₄ getrocknet und das Lösungsmittel im Vakuum entfernt. Das Produkt lag in Form eines hellgelben Feststoffes vor.

### Ausbeute: 91%

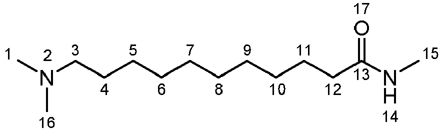

¹H-NMR (400 MHz, CDCl₃): δ [ppm] = 7,26 (CDCl₃); 5,50 (brs, 1H, NH); 2,80 (s, 3H, H-15); 2,33 (t, 2H, J = 7,7 Hz, H-3); 2,29 (s, 6H, H-1 und H-16); 2,15 (t, 2H, J = 7,6 Hz, H-12); 1,65 - 1,57 (m, 2H, H-11); 1,53 - 1,46 (m, 2H, H-4) und 1,34 - 1,22 (m, 12H, H-5 bis H-10).

### Referenzbeispiel 11

### Darstellung des N,N,N'-Trimethylundecan-1,11-diamins

LiAlH₄ (2 M in trockenem THF, 2,7 mL, 5,40 mmol) in trockenem THF (5 mL) wurde in einem Eisbad auf -2 °C abgekühlt. Bei dieser Temperatur wurde eine Lösung von 11-(Dimethylamino)-N-methylundecanamid (660 mg, 2,72 mmol) in trockenem THF (15 mL) langsam zugetropft. Während der Zugabe wurde die Lösung trüb. Nach Zugabe wurde die Reaktionsmischung langsam auf Raumtemperatur erwärmt und im Anschluss unter Rückfluss für drei Stunden erhitzt. Die Reaktionsmischung wurde im Anschluss auf 0 °C abgekühlt und vorsichtig mit H₂O (205 µL), 15%iger NaOH (205 µL) und erneut H₂O (615 µL) gequencht, wobei die anorganischen Salze ausfielen. Die Suspension wurde für weitere 30 Minuten bei Raumtemperatur gerührt. Um den Feststoff abzutrennen, wurde die Suspension zentrifugiert und der Feststoff mehrfach mit trockenem THF gewaschen. Die vereinigten organischen Phasen wurden über Na₂SO₄ getrocknet und das Lösungsmittel wurde im Vakuum entfernt. Das Rohprodukt lag in Form eines gelben Öls vor und wurde ohne weitere Aufarbeitung eingesetzt.

### Ausbeute: 96% (Rohausbeute)

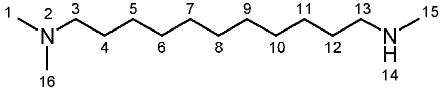

¹H-NMR (600 MHz, CDCl₃): δ [ppm] = 7,26 (CDCl₃); 2,55 (t, 2H, J = 7,2 Hz, H-13); 2,43 (s, 3H, H-15); 2,23 (t, 2H, J = 7,4 Hz, H-3); 2,21 (s, 6H, H-1 und H-16); 1,51 - 1,43 (m, 4H, H-4 und H-12) und 1,34 - 1,23 (m, 14H, H-5 bis H-11).

### Referenzbeispiel 12

### Darstellung des tert-Butyl(11-(dimethylamino)undecyl(methyl)carbamats

Di-*tert*-butyldicarbonat (512 µL, 2,23 mmol) und Amberlyst 15 (76 mg, 15 Gew.%) wurden in einem Rundkolben vorgelegt. Im Anschluss wurde N,N,N'-Trimethylundecan-1,11-diamin (505 mg, 2,21 mmol) hinzugetropft und es wurde eine starke Gasentwicklung beobachtet. Die Reaktionsmischung wurde 15 Minuten gerührt, anschließend mit DCM (22 mL) verdünnt und das Amberlyst abfiltriert. Das Lösungsmittel wurde am Rotationsverdampfer entfernt und das Rohprodukt säulenchromatographisch (DCM/ Methanol/ TEA) gereinigt. Das Produkt lag in Form eines hellgelben Öls vor.

### Ausbeute: 57%

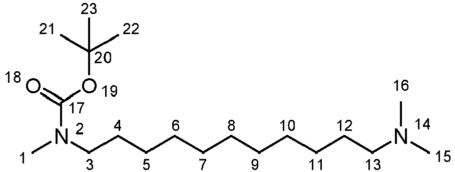

¹H-NMR (400 MHz, CDCl₃): δ [ppm] = 7,26 (CDCl₃); 3,17 (t, 2H, J = 6,9 Hz, H-3); 2,82 (s, 3H, H-1); 2,27 (t, 2H, J = 7,6 Hz, H-13); 2,24 (s, 6H, H-15 und H-16); 1,52 - 1,46 (m, 4H, H-4 und H-12); 1,45 (s, 9H, H-21, H-22 und H-23) und 1,28 - 1.26 (m, 14H, H-5 bis H-11).

¹³C-NMR (100 MHz, CDCl₃): δ [ppm] = 156.0 (C-17); 79,2 (C-20); 77,2 (CDCl₃); 60,0 (C-13); 48,7 (C-3); 45,5 (C-15 und C-16); 34,2 (C-1); 29,7 - 29,5 (C-5 bis C-11); 28,6 (C-21, C-22 und C-23); 27,8 - 27,6 (C-4 und C-12) und 26,9 (C-5 bis C-11).

ESI-MS: (m/z) ber. für C₁₉H₄₁N₂0₂ (M + H)⁺ 329,3163, gef. 329,3169.

IR (neat): u [cm⁻¹] = 2973; 2925; 2854; 1696 (C=O) und 1153 (C-O).

### Referenzbeispiel 13

### Darstellung des 3-((11-((tert-Butoxycarbonyl)(methyl)amino)undecyl)-dimethylammonio)-propan-1-sulfonats

*tert*-Butyl-(11-(dimethylamino)undecyl(methyl)carbamat (250 mg, 0,76 mmol) und 1,3-Propansulton (105 µL, 1,17 mmol) wurden in trockenem DCM (10 mL) gelöst. Die Reaktionsmischung wurde 11 Tage bei Raumtemperatur gerührt. Das Lösungsmittel wurde weitestgehend am Rotationsverdampfer entfernt und der Überschuss 1,3-Propansulton durch mehrmaliges Waschen mit trockenem THF abgetrennt. Der Rückstand wurde im Vakuum getrocknet und das Produkt lag als farbloser Feststoff vor.

### Ausbeute: 77%

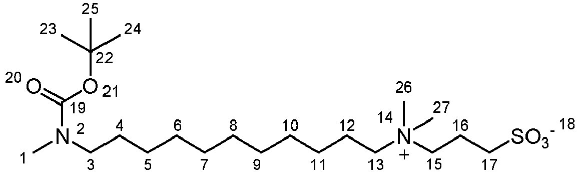

¹H-NMR (600 MHz, D₂O): δ [ppm] = 4,79 (D₂O); 3,50 - 3,47 (m, 2H, H-15); 3,35 - 3,32 (m, 2H, H-13); 3,24 - 3,25 (m, 2H, H-3); 3,12 (s, 6H, H-26 und H-27); 2,99 (t, 2H, J = 7,1 Hz, H-17); 2,84 (s, 3H, H-1); 2,26 - 2,20 (m, 2H, H-16); 1,79 - 1,77 (m, 2H, H-12); 1,57 - 1,53 (m, 2H, H-4); 1,46 (s, 9H, H-23, H-24 und H-25); 1,38 (m, 4H, H-10 und H-11); 1,33 (m, 8H, H-6 bis H-9) und 1,30 - 1,28 (m, 2H, H-5).

¹³C-NMR (150 MHz, D₂O): δ [ppm] = 156,8 (C-19); 81,1 (C-22); 65,1 (C-13); 62,9 (C-15); 51,5 (C-26 und C-27); 49,4 (C-3); 48,2 (C-17); 34,5 (C-1); 29,9 - 29,3 (C-5 bis C-10); 28,8 (C-23, C-24 und C-25); 26,9 (C-4); 26,5 (C-12); 22, 8 (C-11) und 19,0 (C-16).

ESI-MS: (m/z) ber. für C₂₂H₄₆N₂O₅S (M + H)⁺ 451,3200, gef. 451,3214.

IR (neat): u [cm⁻¹] = 3456; 2973; 2925; 2855; 1680 (C=O); 1165 (C-O); 1035 (S=O), 606 und 521.

### Referenzbeispiel 14

### Darstellung des 3-(Dimethyl(11-(methylamino)undecyl)ammonio)propan-1-sulfonats

3-((11-((*tert*-Butoxycarbonyl)(methyl)amino)undecyl)dimethylammonio)-propan-1-sulfonat (600 mg, 1,33 mmol) wurde in Reinstwasser (13 mL) gelöst. Das Reaktionsgefäß wurde mit Stickstoff gespült und die Lösung wurde für 90 Minuten in der Mikrowelle auf 135 °C erhitzt. Das Wasser wurde durch Lyophilisation entfernt und das Produkt lag als farbloser, stark hygroskopischer Feststoff vor.

### Ausbeute: 93%

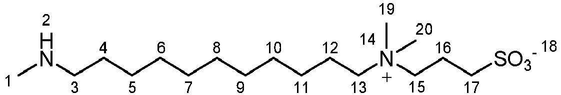

¹H-NMR (400 MHz, D₂O): δ [ppm] = 4,79 (D₂O); 3,51 - 3,46 (m, 2H, H-15); 3,36 - 3,32 (m, 2H, H-13); 3,12 (s, 6H, H-19 und H-20); 3,01 (t, 2H, J = 7,2 Hz, H-17); 2,95 - 2,91 (m, 2H, H-3); 2,63 (s, 3H, H-1); 2,28 - 2,20 (m, 2H, H-16); 1,81 - 1,77 (m, 2H, H-12); 1,69 - 1,61 (m, 2H, H-4); 1,39 (m, 4H, H-5 und H-11) und 1,33 (m, 10H, H-6 bis H-10).

¹³C-NMR (100 MHz, D₂O): δ [ppm] = 64,2 (C-13); 61,9 (C-15); 50,6 (C-19 und C-20); 49,5 (C-3); 47,3 (C-17); 33,0 (C-1); 28,4 - 28,1 (C-6 bis C-10); 26,0 (C-4); 25,7 - 25.4 (C-5 und C-11); 21,7 (C-12) und 18,1 (C-16).

### ESI-MS: (m/z) ber. für C₁₇H₃₉N₂O₃S (M + H)⁺ 351,2676, gef. 351,2689.

IR (neat): u [cm⁻¹] = 2919; 2849; 1196 (S=O); 1036 (S=O), 603 und 526.

### Referenzbeispiel 15

### Dithiocarbamat-Bildung

Die Dithiocarbamat-Bildung wurde absorptionsspektroskopisch im Bereich von 230-400 nm verfolgt. Dazu wurden 1 mL einer 25 mM Lösung des Amins gemäß Beispiel 3 bzw. Beispiel 14 und 500 µL einer 0,66 M Lösung CS₂ in MeOH angesetzt. Für die Referenzmessungen der Ausgangssubstanzen wurden je 10 µL der Stammlösungen mit 1.5 mL MeOH in einer UV-Halbmikroküvette verdünnt und die Absorption gemessen. Zur Verfolgung der Dithiocarbamat-Bildung wurden 38 µL der CS₂-Lösung zur restlichen Aminlösung gegeben und die Absorption über einen Zeitraum von 40 bzw. 110 Minuten verfolgt. Dazu wurden 10 µL der Reaktionsmischung nach unterschiedlichen Zeitintervallen mit 1,5 mL MeOH verdünnt und am Absorptionsspektrometer vermessen. Sobald die charakteristischen Absorptionsmaxima bei 255 und 291 nm nicht mehr zunahmen, war die Dithiocarbamat-Bildung abgeschlossen und die Ligandenlösung wurde im nächsten Schritt eingesetzt.

### Referenzbeispiel 16

### Ligandenaustausch

Vor dem Ligandenaustausch wurden die QDQRs (1 nmol) drei Mal mit MeOH gefällt und bei 6.700 x g zentrifugiert. Der farblose Überstand wurde verworfen und die Nanopartikel in 2,5 mL n-Hexan aufgenommen. Des Weiteren wurden Verdünnungen der zwitterionischen Amine gemäß Beispiel 3, Beispiel 14, Beispiel 6 und Beispiel 8 (je 0,2 M) sowie des CS₂ (0,66 M) in MeOH angesetzt. Die Ligandenüberschüsse variierten zwischen 10.000 und 100.000 bezogen auf die Konzentration an Nanopartikeln, wobei das Verhältnis von Amin zu CS₂ 1:1 betrug. Zur Bildung der Dithiocarbamat-Liganden wurden die entsprechenden Mengen Amin und CS₂ auf ein Gesamtvolumen von 1 mL mit MeOH aufgefüllt und 15 Minuten (für die Amine gemäß Beispiel 3, 14 und 8) bzw. 90 Minuten (für das Amin Beispiel 6) bei Raumtemperatur gerührt. Im Anschluss wurde die Ligandlösung zu den Nanopartikeln in n-Hexan gegeben und das Zweiphasensystem wurde 30 Minuten kräftig gerührt, bis die QDQRs aus der Reaktionslösung ausfielen. In manchen Fällen war eine verlängerte Reaktionszeit von bis zu sechs Stunden notwendig, um eine Fällung der Nanopartikel zu erreichen. Nach kurzer Zentrifugation (1 Minute bei 1.000 x g) wurden die nun farblosen organischen Phasen entfernt, die Nanopartikel im Stickstoffstrom vorsichtig getrocknet und anschließend in Reinstwasser (0,5 - 1 mL) aufgenommen.

### Referenzbeispiel 17

### Stabilitätsmessungen

Für die Stabilitätsmessungen wurden folgende pH-Puffer angesetzt:
Citrat-Puffer pH 5,1

Citronensäure (960 mg, 5 mmol) wurde in einem 50 mL Maßkolben abgewogen, in Reinstwasser (ca. 40 mL) gelöst und mit Hilfe einer 1 M NaOH auf einen pH Wert von 5,1 eingestellt. Anschließend wurde bis zur Markierung mit Reinstwasser aufgefüllt (Konzentration: 0,1 M).

### Phosphat-Puffer pH 7,4

PBS-Puffer mit einem pH Wert von 7,4 wurde kommerziell erworben.

### Borat-Puffer pH 9,0

H₃BO₃ (62 mg, 1 mmol) wurde in einem 50 mL Maßkolben abgewogen, in Reinstwasser (ca. 40 mL) gelöst und mit Hilfe einer 1 M NaOH auf einen pH Wert von 9,0 eingestellt. Anschließend wurde bis zur Markierung mit Reinstwasser aufgefüllt (Konzentration: 20 mM).

### Phosphat-Puffer pH 11,5

K₂HPO₄ (8,71 g, 50 mmol) wurde in einem 50 mL Maßkolben abgewogen und in wenig Reinstwasser gelöst. Anschließend wurde bis zur Markierung mit Reinstwasser aufgefüllt (Konzentration: 1 M). Von dieser Lösung wurden 500 µL mit 49,5 mL Reinstwasser verdünnt (Konzentration 0,01 M) und mit Hilfe einer 1 M NaOH wurde ein pH Wert von 11,5 eingestellt.

Des Weiteren wurden für die Stabilitätsmessungen folgende Medien verwendet: 10 mM HEPES, DMEM (ohne Phenolrot), 10% FCS in DMEM, PBS und 1% BSA in PBS.

Für die Stabilitätsmessungen wurden 990 µL des pH-Puffers oder Mediums in einer Quarzküvette vorgelegt und 10 µL der Nanopartikellösung hinzugefügt (Endkonzentration: 20 nM). Nach einer Inkubationszeit von zwei Minuten wurde die Fluoreszenzintensität der Proben innerhalb der ersten zwei Stunden alle 20 Minuten, im weiteren Verlauf des ersten Tages stündlich und im Anschluss daran alle 24 Stunden gemessen. Insgesamt wurde die Fluoreszenzintensität über einen Zeitraum von einer Woche verfolgt. Die Messungen wurden bei einer Anregungswellenlänge von 350 nm durchgeführt.

### Referenzbeispiel 18

### Zellkulturexperimente

Das Kultivierungsmedium bestand aus 10% FCS in DMEM mit je 1% PenStrep und Natriumpyruvat. Die Inkubation der Zellen fand stets bei 37 °C und einem CO₂-Gehalt von 5% statt.

Die zwitterionischen QuantumDots/QuantumRods(QDQRs) Nanopartikel wurden in diversen *in vitro* Versuchen hinsichtlich ihrer Toxizität und der unspezifischen Zellaufnahme untersucht. Für die Versuche wurden gelb-emittierende QDQRs mit dem kurz- und langkettigen zwitterionischen Liganden (QDQRs-17a und QDQRs-17b) verwendet, um einen möglichen Einfluss der Kettenlänge des Liganden auf die Wechselwirkungen der QDQRs mit Zellen zu untersuchen. Sowohl für die Toxizitätsuntersuchungen als auch für die Zellaufnahmestudien wurden A549 Zellen verwendet. A549 Zellen sind humane, aus einem Adenokarzinom der Lunge stammende basale Epithelzellen, die als Zelllinie kultiviert wurden.

Für den Einsatz von Nanopartikeln in der Biochemie oder Medizin ist es unabdingbar, dass von ihnen keine intolerable Toxizität ausgeht. Generell besteht bei cadmiumhaltigen Nanopartikeln die Möglichkeit, dass die Zellen durch austretende Cadmiumionen geschädigt werden. Die Wahrscheinlichkeit, dass Cadmiumionen in die Umgebung freigesetzt werden, ist gerade bei den hier verwendeten QDQRs relativ hoch. Zum einen ist der Anteil an Cadmium in den elongierten Nanopartikeln im Vergleich zu sphärischen Partikeln gleichen Durchmessers um den Faktor 12 höher. Zum anderen fehlt den QDQRs eine passivierende Schale aus Zinksulfid, mit der cadmiumhaltige Nanopartikel oftmals umhüllt werden, um das Herauslösen von Cadmiumionen zu verhindern.

Die Toxizität der zwitterionischen QDQRs-17a und QDQRs-17b wurde mit Hilfe des Cellomics Array Scan geprüft. Für die Toxizitätsuntersuchungen wurden A549-Zellen auf einer 96-Well Platte ausplattiert und mit QDQR-Lösungen in einem Konzentrationsbereich von 25 bis 500 nM inkubiert. Als Positivkontrolle wurde Cadmiumchlorid verwendet, welches in einem Konzentrationsbereich von 25 bis 1.000 µM eingesetzt wurde. Die um den Faktor 1.000 höhere Konzentration an Cadmiumchlorid ist der Tatsache geschuldet, dass in einem einzelnen QDQR der untersuchten Proben etwa 8.200 Cadmiumatome enthalten sind. Unter der Annahme, dass sich die gesamten Nanopartikel auflösen und das in ihnen enthaltene Cadmium freisetzen, entspräche die kleinste eingesetzte Konzentration von 25 nM QDQRs einer Konzentration von 205 µM Cadmiumchlorid. Nach einer Inkubationszeit von 16 Stunden wurden die Zellen mit einer Färbelösung, bestehend aus dem Zellkernfarbstoff Hoechst 33342 und dem Mitochondrienfarbstoff MitoTracker^{®} Deep Red FM, versetzt und danach mit Formaldehydlösung fixiert. Im Anschluss wurden die 96-Well Platten mit dem Cellomics Array Scan ausgelesen. Dabei wurden anhand der Intensität des Hoechstfarbstoffs die Parameter Zellzahl, Zellkernintensität sowie Zellkerngröße und anhand des Mitochondrienfarbstoffs das Transmembranpotential der Mitochondrien mit Hilfe einer Software automatisch bestimmt. Des Weiteren wurden mikroskopische Bilder der Zellen zur Kontrolle der Zellzahl und Zellmorphologie aufgenommen.

### Referenzbeispiel 19

### Toxizitätstests

Für die Toxizitätstests wurden A549-Zellen in Kultivierungsmedium auf einer 96-Well Platte ausplattiert (10.000 Zellen pro Well) und über Nacht inkubiert. Nach Absaugen des Mediums wurden die zu testenden Proben (verdünnt mit Kultivierungsmedium, 100 µL pro Well) in unterschiedlichen Konzentrationen aufgetragen und für 16 Stunden inkubiert. Im Anschluss wurden die Proben entfernt und die Zellen mit 100 µL Färbelösung (Endkonzentration: 75 nM MitoTracker^{®} Deep Red und 2,5 µg/ml Hoechst 33342 in DMEM mit 10% FCS) versetzt und weitere 30 Minuten inkubiert. Zur Fixierung der Zellen wurde die Färbelösung entfernt und 100 µL einer warmen Fixierlösung (3,7% Formaldehyd in Dulbecco's PBS, DPBS) hinzugegeben. Nach 20 Minuten wurde die Fixierlösung abgesaugt und zwei Mal mit DPBS gewaschen, bevor die Proben am Cellomics Array Scan vermessen wurden.

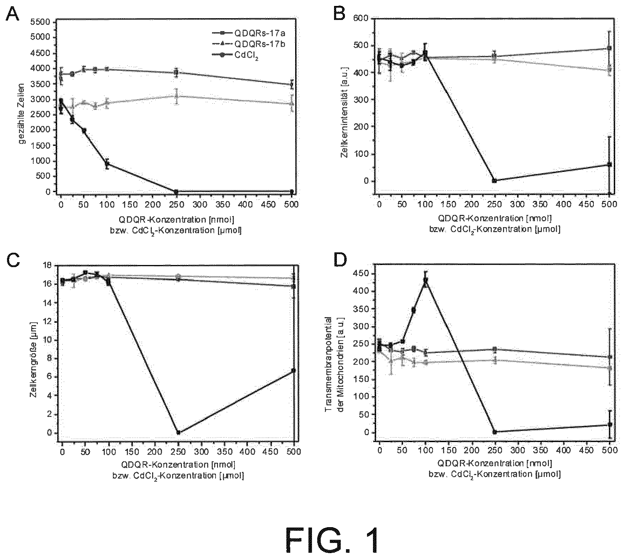

Figur 1 zeigt eine Quantifizierung der Toxizitätstests der QDQRs-17a und QDQRs-17b sowie Cadmiumchlorid (mit (A) Anzahl der Zellen, (B) Intensität des Zellkernfarbstoffs Hoechst 33342, (C) Größe der Zellkerne und (D) Transmembranpotential der Mitochondrien; Anmerkung: Da Cadmiumchlorid bereits ab einer Konzentration von 100 µM eine toxische Wirkung auf die Zellen zeigt, sind in der Darstellung nur die Werte bis 500 µM abgebildet).

In Figur 1 sind die oben genannten Parameter in Abhängigkeit der eingesetzten Konzentration an QDQRs bzw. Cadmiumchlorid gezeigt. Jeder Datenpunkt stellt dabei den Mittelwert aus drei Messwerten dar. Anhand der Diagramme A bis D in Figur 1 wird deutlich, dass sowohl QDQRs-**17a** als auch QDQRs-**17b** einschließlich der höchsten getesteten Konzentration von 500 nM keine erkennbare toxische Wirkung auf die Zellen haben. Die Anzahl der Zellen pro Well (A), die Zellkernintensität (B), die Zellkerngröße (C) und auch das Transmembranpotential der Mitochondrien (D) bleiben über den gesamten Konzentrationsbereich nahezu konstant.

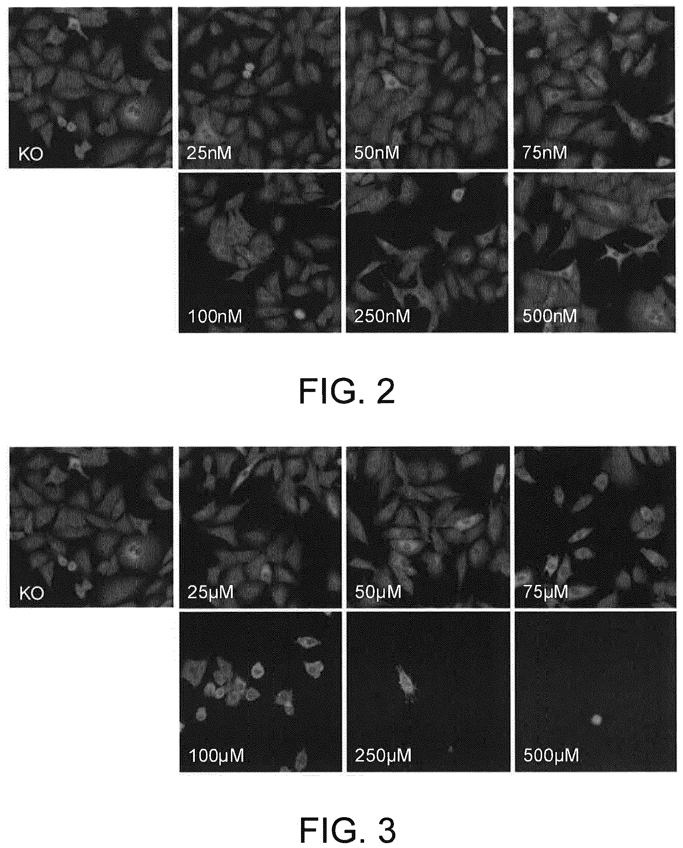

Ebenso sind bei der Betrachtung der mikroskopischen Aufnahmen keine morphologischen Änderungen der Zellen oder Zellkerne nach 16-stündiger Inkubation mit den zwitterionischen QDQRs erkennbar. Als Beispiel hierfür sind in Figur 2 die mikroskopischen Aufnahmen der A549-Zellen nach Inkubation mit QDQRs-**17a** dargestellt. Die Eigenfluoreszenz der QDQRs ist in den Bildern nicht zu sehen, da sie durch entsprechende Filtereinstellungen am Gerät unterdrückt wurde.

Figur 2 zeigt Mikroskopische Aufnahmen der A549-Zellen nach 16-stündiger Inkubationszeit mit unterschiedlichen Konzentrationen von QDQRs-17a. Die Zellkerne sind mit Hoechst 33342 (blau) und die Mitochondrien mit MitoTracker^{®} Deep Red (rot) angefärbt. Zur Kontrolle (KO) ist eine Aufnahme von A549-Zellen nach 16 Stunden gezeigt, die ohne QDQRs inkubiert wurden.

Im Gegensatz dazu ist bei Cadmiumchlorid ab einer Konzentration von 100 µM, die etwa einer QDQR-Konzentration von 12 nM entspricht, eine toxische Wirkung auf die Zellen erkennbar. Die Anzahl an Zellen pro Well, die Zellkernintensität und die Zellkerngröße nimmt signifikant ab, was durch das Absterben der Zellen bedingt ist (Figur 1 A - C). Das Transmembranpotential steigt im Konzentrationsbereich von 50 bis 100 µM erst an und fällt dann wieder ab (Figur 1 D). Dieser Verlauf ist typisch für die toxische Wirkung einer Substanz. Durch Zellstress werden Apoptose-auslösende Faktoren freigesetzt und es kommt zunächst zu einer Zunahme der Mitochondrienmasse, was in der Messung zu einer Intensitätssteigerung des Mitochondrienfarbstoffs führt. Bei weiterer Erhöhung der Cadmiumchlorid-Konzentration sterben die Zellen zunehmend ab, wodurch die Mitochondrienmasse ebenfalls abnimmt und das Signal des Farbstoffs wieder schwächer wird. Die erneute Zunahme der Zellkernintensität und der Zellkerngröße bei einer Konzentration von 500 µM Cadmiumchlorid sowie die großen Fehlerbalken dieser Messwerte sind dadurch zu erklären, dass bei hoher Toxizität einer Substanz nur noch sehr wenige Zellen im Well vorhanden sind, wodurch weniger Messpunkte für die Auswertung zur Verfügung stehen und die statistischen Fehler entsprechend groß werden.

Besonders deutlich wird dies bei der Betrachtung der mikroskopischen Aufnahmen der Zellen nach 16-stündiger Inkubation mit Cadmiumchlorid, die in Figur 3 dargestellt sind. Ab einer Konzentration von 100 µM Cadmiumchlorid sinkt die Anzahl an Zellen merklich und sie kugeln sich ab. Bei höheren Konzentrationen Cadmiumchlorid sind in den Wells nur noch vereinzelt Zellen zu finden.

Figur 3 zeigt mikroskopische Aufnahmen der A549-Zellen nach 16-stündiger Inkubationszeit mit unterschiedlichen Konzentrationen Cadmiumchlorid. Die Zellkerne sind mit Hoechst 33342 (blau) und die Mitochondrien mit MitoTracker^{®} Deep Red (rot) angefärbt. Zur Kontrolle (KO) ist eine Aufnahme von A549-Zellen nach 16 Stunden gezeigt, die ohne Cadmiumchlorid inkubiert wurden.

Zusammenfassend lässt sich sagen, dass trotz des hohen Cadmiumgehalts in den verwendeten QDQRs und dem Fehlen einer zusätzlich passivierenden Zinksulfidhülle keine messbare Toxizität von den QDQRs bis zu einer Konzentration von 500 nM ausgeht. Damit erfüllen die hergestellten zwitterionischen QDQRs eine wichtige Voraussetzung für spätere biomedizinische Anwendungen.

So konnte gezeigt werden, dass die zwitterionischen QDQRs auch in hohen Konzentrationen von bis zu 500 nM keine toxische Wirkung auf A549-Zellen haben.

Anschließend wurde das Aufnahmeverhalten der QDQRs-**17a** und QDQRs-**17b** mit A549-Zellen analysiert und überprüft, ob die Nanopartikel in Abhängigkeit der Kettenlänge des Liganden unterschiedlich aufgenommen werden.

### Beispiel 20

### Zellaufnahmeversuche mit A549-Zellen (in-vitro)

Für die Zellaufnahmeversuche wurden A549-Zellen in Kultivierungsmedium auf einem 8-Well Labtek ausplattiert (20.000 Zellen pro Well) und 24 bzw. 48 Stunden inkubiert.

Das Medium wurde abgesaugt und die zu untersuchenden Proben (verdünnt mit DMEM und entsprechend mit oder ohne 10% FCS, Konzentration: 100 nM) hinzugegeben. Im Anschluss wurden die Zellen für vier bzw. 16 Stunden inkubiert. Nach Ablauf der Inkubationszeit wurde das Medium entfernt, die Zellen mit einer Färbelösung (Endkonzentration: 2,5 µg/ml Hoechst 33342 in DMEM, 200 µL pro Well) versetzt und erneut für 30 Minuten inkubiert. Die Fixierung der Zellen erfolgte ebenfalls mit einer 3,7%igen Formaldehyd-Lösung in DPBS für 20 Minuten. Nach zweimaligem Waschen der Zellen mit DPBS wurden die Proben am Konfokalmikroskop vermessen.

Es wurden zum einen zwei unterschiedlich lange Inkubationszeiten (vier und 16 Stunden) getestet. Zum anderen wurde die Inkubation entweder in Gegenwart oder Abwesenheit von 10% FCS durchgeführt. Durch die Abwesenheit von FCS im Medium sollte untersucht werden, ob die Nanopartikelaufnahme gegebenenfalls in die hungernden Zellen forciert werden kann. In allen Experimenten wurden die Zellen mit einer 100 nM QDQR-Lösung inkubiert. Nach der Inkubation wurden die Zellen gewaschen, die Zellkerne mit Hoechst 33342 gefärbt, fixiert und mit Hilfe eines Konfokalmikroskops analysiert.

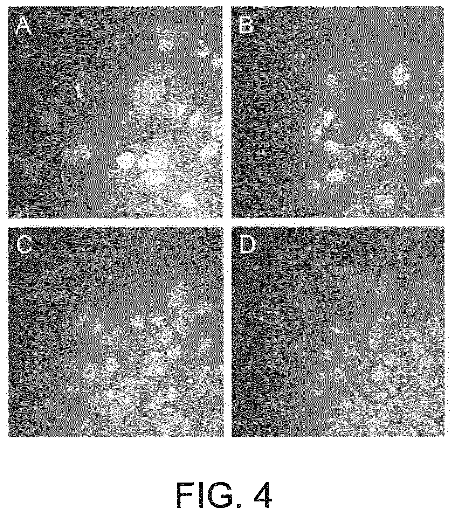

Für QDQRs-**17a** war unter allen experimentellen Bedingungen eine unspezifische Aufnahme der Nanopartikel zu erkennen, siehe Figur 4. Die Aufnahme nach vier Stunden (A und B) war dabei nicht so stark ausgeprägt wie nach 16 Stunden (C und D). Nach vierstündiger Inkubation befanden sich die QDQRs zudem vornehmlich in der Nähe der Zellmembran, wohingegen die Nanopartikel nach längerer Inkubationszeit mehr oder weniger gleichmäßig im Zellinneren verteilt waren. Die Fluoreszenz der Nanopartikel war dabei oftmals in punktförmigen Strukturen zu erkennen, bei denen es sich mutmaßlich um mit QDQRs gefüllte Endosomen handelt. Da A549-Zellen keine phagozytierenden Zellen sind, gelangen die QDQRs höchstwahrscheinlich über unspezifische Endozytose in die Zellen. Außerdem wurden die QDQRs nach vier Stunden Inkubationszeit in Gegenwart von 10% FCS (B) im Vergleich zum Experiment unter serumfreien Bedingung (A) deutlich weniger von den Zellen aufgenommen. Eine reduzierte Aufnahme von Nanopartikeln in serumhaltigem Medium ist bereits aus der Literatur bekannt, wobei die verringerte Aufnahme auf das Vorhandensein einer Proteinkorona zurückgeführt wird. Obwohl zwitterionische Liganden auf Nanopartikeln die Bildung einer Proteinkorona verhindern sollen, scheinen die zwitterionischen QDQRs-**17a** mit dem Protein in Lösung zu wechselwirken, was in einer verringerten Zellaufnahme resultiert.

Figur 4 zeigt Konfokalmikroskopische Aufnahmen von A549-Zellen nach der Inkubation mit 100 nM QDQRs-17a für vier Stunden (A und B) und 16 Stunden (C und D). Die Inkubation erfolgte unter serumfreien Bedingungen (A und C) oder in Gegenwart von 10% FCS (B und D). Die Zellkerne wurden mit dem Farbstoff Hoechst 33342 angefärbt (blau). Die roten Bereiche resultieren aus der Fluoreszenz der QDQRs.

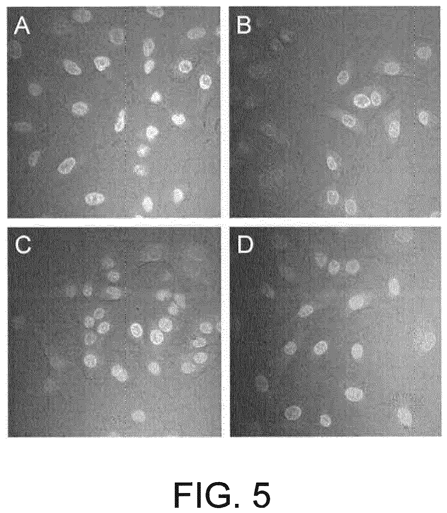

Bei der Inkubation von A549-Zellen mit QDQRs-**17b** zeigte sich überraschenderweise ein völlig anderes Bild (siehe Figur 5).

Figur 5 zeigt konfokalmikroskopische Aufnahmen von A549-Zellen nach der Inkubation mit 100 nM QDQRs-17b für vier Stunden (A und B) und 16 Stunden (C und D). Die Inkubation erfolgte unter serumfreien Bedingungen (A und C) oder in Gegenwart von 10% FCS (B und D). Die Zellkerne wurden mit dem Farbstoff Hoechst 33342 angefärbt (blau). Die roten Bereiche resultieren aus der Fluoreszenz der QDQRs.

Die Nanopartikel wurden nach vierstündiger Inkubationszeit weder unter serumhaltigen (B) noch unter serumfreien Bedingungen (A) von den Zellen aufgenommen. Auch die erhöhte Inkubationszeit von 16 Stunden führte in Gegenwart von 10% FCS zu keiner unspezifischen Aufnahme der QDQRs (D). Lediglich in serumfreiem Medium war eine leichte unspezifische Aufnahme der QDQRs zu erkennen (C), die jedoch im Vergleich zu QDQRs-**17a** deutlich geringer ausfiel.

Allgemein ist Zellaufnahme von Nanopartikeln von vielen verschiedenen Faktoren, wie z. B. der Größe und Form der Partikel, ihrer Oberflächenladung, oder dem Vorhandensein einer Proteinkorona abhängig. Wie anhand der DLS-Messungen und der TEM-Aufnahmen gezeigt werden konnte, sind sowohl die Größe als auch die Form von QDQRs-**17a** und QDQRs-**17b** nahezu identisch, so dass diese Faktoren das unterschiedliche Zellaufnahmeverhalten nicht erklären können.

Zusammenfassend lässt sich sagen, dass sich die QDQRs überraschend deutlich in ihrem Aufnahmeverhalten von A549-Zellen unterscheiden. Somit sind die zwitterionischen Liganden auf der Nanopartikeloberfläche geeignet, die Zellaufnahme durch Strukturveränderungen zu beeinflussen.

### In vivo Untersuchungen

Für die *in vivo* Experimente wurden 12 Wochen alte FVB Mäuse durch Inhalation von 4%igem Isofluran narkotisiert. Während der Experimente wurde die Narkose durch Inhalation von 1-1,5%igem Isofluran aufrechterhalten. Für die Intravital-Mikroskopie wurden unter Narkose die Leber und der Darmtrakt der Maus freigelegt und an einem Deckgläschen befestigt. Um eine Hypothermie der Maus zu vermeiden, wurden die Experimente in einer klimatisierten Kammer bei einer Temperatur von 32 °C durchgeführt. Die Visualisierung der Nanopartikelaufnahme erfolgte mit Hilfe eines Konfokalmikroskops, das mit einem resonanten Scanner (Nikon A1R) ausgestattet war. Der Maus wurden intravenös 150 µL einer 10 µM QDQR-Lösung in steriler, physiologischer NaCl verabreicht und die Aufnahme der Nanopartikel wurde mit einer Abtastrate von 30 Bildern pro Sekunde verfolgt. Zusätzlich wurden Bilder der Leber vor und nach Injektion der QDQRs aufgenommen. Zur Markierung der Makrophagen wurden grün-emittierende, etwa 1 µm große FluoSpheres^{®} aus Polystyrol (~ 10⁸ Partikel/ Maus) 15 Minuten nach Injektion der QDQRs ebenfalls intravenös verabreicht.

FluoSpheres ^{®} sind Mikrokugeln (auch Latex Beads oder Latex Partikel genannt, die sich in kolloidalen Größenordnungen bewegen und aus einem amorphen Polymer gebildet sind, wie beispielsweise Polystyrol. Zum Verfolgen biologischer Ereignisse können sie mit Chromophoren, insbesondere mit Fluoreszenzfarbstoffen beladen oder markiert sein.

Um die Aufnahme in andere Gewebe zu untersuchen wurde die Maus 15 Minuten nach Injektion der QDQRs mit 4%igem Paraformaldehyd in PBS perfundiert. Nach Entnahme von Lunge, Milz, Niere und interskapularem, braunen Fettgewebe wurden die Gewebe sofort mikroskopisch analysiert.

Für die Immunfärbung wurden nach der Perfusion der Maus 200 µm dicke Leberschnitte mit Hilfe eines VT1000S Mikrotom (Leica) angefertigt. Die Schnitte wurden mit PBS gewaschen und zur Reduktion der Autofluoreszenz für 30 Minuten mit 5% Glycin in PBS inkubiert. Im Anschluss wurden die Schnitte für zwei Stunden in 3% BSA und 0,3% Triton X-100 (jeweils in PBS) blockiert und permeabilisiert. Nach erneutem Waschen der Schnitte wurden diese für 48 Stunden bei 4 °C mit dem Primärantikörper (Ratte-Anti-Maus CD31, 1:500 in 1% BSA) inkubiert. Daraufhin wurde die Schnitte drei Mal für je 10 Minuten mit PBS gewaschen und über Nacht bei 4 °C mit dem Sekundärantikörper (anti-Ratte Cy5, 1:500 in 1% BSA) inkubiert. Die Schnitte wurden erneut drei Mal mit PBS gewaschen und die Zellkerne mit DAPI angefärbt. Die Analyse der Schnitte erfolgte mit Hilfe eines Konfokalmikroskops (Nikon A1R).

### Beispiel 21

### Aufnahme und Organverteilung von QDQRs-17a und QDQRs-17b in vivo

Prinzipiell kann nach intravenöser Injektion von Nanopartikeln zwischen drei verschiedenen Mechanismen unterschieden werden, die die Nanopartikel wieder aus dem Blutkreislauf entfernen. Die Ausscheidung kann durch die Nieren mit dem Urin (renal) oder durch die Leber mit der Galle (hepatobiliär) erfolgen. Darüber hinaus können die Nanopartikel durch Aufnahme in Makrophagen des retikuloendothelialen Systems (RES, Leber, Milz oder Knochenmark) entfernt werden.³ Die Leber ist eines der Hauptorgane, durch das verschiedenste Nanopartikel aufgenommen und gegebenenfalls wieder ausgeschieden werden .^{4,5} In der Leber sind vor allem die sogenannten Kupffer-Zellen, speziell in der Leber vorkommende Makrophagen, für die Aufnahme von größeren Partikeln verantwortlich.^{6,7} Außerdem ist aus der Literatur bekannt, dass neben den Kupffer-Zellen auch die sinusoidalen Leberendothelzellen (*liver sinusoidal endothelial cells,* LSECs*)* Nanopartikel internalisieren können.^{8,9} Daher wurde das Verhalten der zwitterionischen QDQRs in Echtzeit mittels Intravital-Mikroskopie in der Leber von Mäusen verfolgt und anschließend die Organverteilung der Nanopartikel *ex vivo* analysiert. Jeder Maus wurden pro Experiment 1,5 nmol QDQRs in physiologischer Kochsalzlösung (150 µL) in die Schwanzvene injiziert. Unter Berücksichtigung des Blutvolumens einer Maus von ca. 3 mL entspricht diese Menge einer QDQR-Konzentration von etwa 500 nM im Organismus. Bis zu dieser Konzentration konnte in den *in vitro* Untersuchungen keine toxische Wirkung der QDQRs beobachtet werden (vgl. Beispiel 19), weshalb diese Konzentration ebenfalls in den *in vivo* Versuchen verwendet wurde.

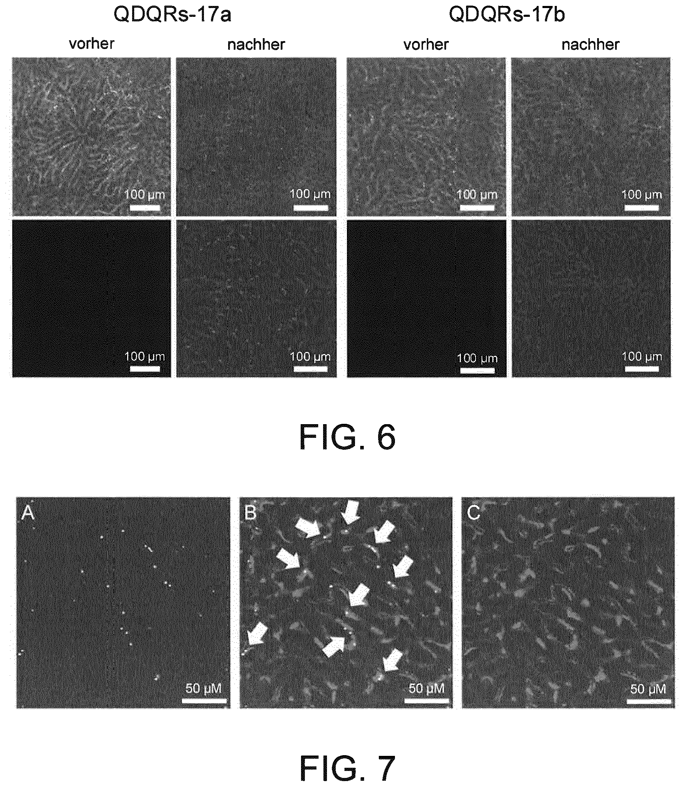

Nach der intravenösen Injektion der QDQRs mit dem kurzkettigen zwitterionischen Liganden **17a** war ein sogenanntes Lining der Blutgefäße in der Leber durch die Nanopartikel zu erkennen, was für eine Aufnahme in LSECs spricht. Zusätzlich zum Lining befanden sich die QDQRs in dichter gepackten, größeren Strukturen, was auf eine Aufnahme in Kupffer-Zellen hindeutet (s. Figur 6**Fehler! Verweisquelle konnte nicht gefunden werden.,** linke Seite). Die QDQRs mit dem langkettigen zwitterionischen Liganden **17b** zeigten ebenfalls ein Lining der Blutgefäße. Im Gegensatz zu QDQRs-**17a** konnten jedoch keine größeren Ansammlungen von Nanopartikeln festgestellt werden (s. Figur 6, rechte Seite). Somit schlägt sich das unterschiedliche Verhalten der QDQRs-**17a** und QDQRs-**17b** *in vitro* auch in den *in vivo* Befunden nieder. Da es in der Leber zur Aufnahme der QDQRs kam, wurde zusätzlich der Darm als stark durchblutetes Organ mit hoher Immunzellaktivität intravital-mikroskopisch untersucht. Im Gegensatz zur Leber kam es im Darm zu keiner unspezifischen Aufnahme der Nanopartikel.

Figur 6 zeigt mikroskopische Aufnahmen der Leber vor und nach der Injektion von QDQRs-17a (linke Seite) und QDQRs-17b (rechte Seite). Obere Reihe: Überlagerung von Hintergrundsignal und dem Kanal, in dem die Fluoreszenz der QDQRs (rote Bereiche) gemessen wird. Untere Reihe: Kanal, in dem die Fluoreszenz der QDQRs gemessen wird.

### Beispiel 22

### Co-Injektion von QDQRs-17a und QDQRs-17b und FluoSpheres^{®} in vivo

Um Aufschluss über die größeren Strukturen in der Leber zu erhalten, in denen sich QDQRs **17a** ansammelten, wurden in einem weiteren Experiment nach Injektion der QDQRs etwa 1 µm große FluoSpheres^{®} injiziert. Aufgrund ihrer Größe akkumulieren die ebenfalls fluoreszierenden Partikel ausschließlich in Phagozyten wie den Kupffer-Zellen, aber nicht in LSECs.¹⁰ Figur 7 zeigt eine mikroskopische Aufnahme eines Leberausschnitts nach Injektion von QDQRs-**17a** und der FluoSpheres^{®}. Die Fluoreszenz der FluoSpheres^{®} ist dabei in Grün (A) und die Fluoreszenz der QDQRs in Rot (C) dargestellt. Figur 7 B zeigt die Überlagerung der beiden Bilder. Es ist deutlich eine Kolokalisation der QDQRs und der FluoSpheres^{®} innerhalb der gleichen Strukturen zu erkennen, was für eine Aufnahme der QDQRs-**17a** in die Kupffer-Zellen der Leber spricht.

Figur 7 zeigt mikroskopische Aufnahme eines Leberausschnittes nach Injektion von QDQRs-17a und FluoSpheres^{®}. Fluoreszenz der FluoSpheres^{®} (A), Überlagerung der Fluoreszenz von FluoSpheres^{®} und QDQRs-17a (B) und Fluoreszenz der QDQRs-17a (C). Die Pfeile in (B) markieren die Strukturen, in denen sich sowohl FluoSpheres^{®} als auch QDQRs-17a befinden.

### Beispiel 23

### Immunhistologische Untersuchung von Gewebsschnitten ex vivo

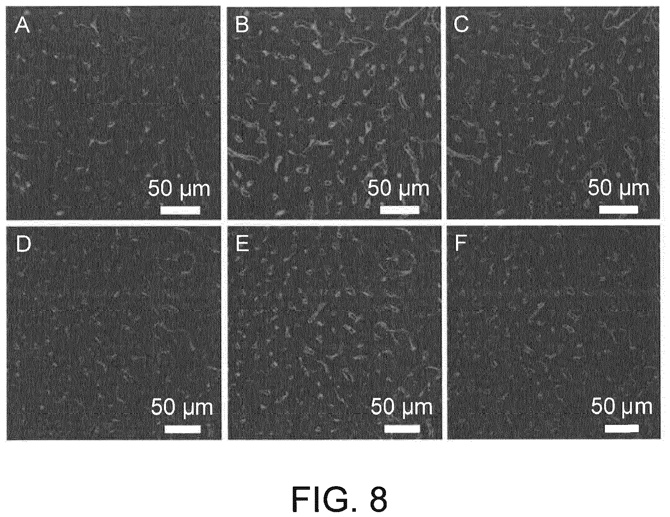

Das Lining der Blutgefäße nach Injektion von QDQRs-**17a** und QDQRs-**17b** lässt, wie schon erwähnt, auf eine Aufnahme der Nanopartikel in LSECs schließen. Um diese Annahme zu bestätigen, wurden die Mäuse nach den Experimenten perfundiert und Vibratomschnitte der Leber angefertigt, die immunohistologisch untersucht wurden. Dazu wurden die Schnitte zunächst mit einem Primärantikörper (Ratte-anti-Maus CD31) und im Anschluss mit einem Sekundärantikörper (anti-Ratte Cy5) inkubiert, um das Leberendothel zu markieren. Zusätzlich wurden die Zellkerne mit dem Fluoreszenzfarbstoff 4',6-Diamidin-2-phenylindol (DAPI) angefärbt. Die Analyse der Leberschnitte erfolgte mit Hilfe eines Konfokalmikroskops. Figur 8 A - C zeigt mikroskopische Aufnahmen eines Leberschnittes einer Maus, der QDQRs-**17a** injiziert wurden. Die Fluoreszenz der QDQRs ist in Rot (A) und der Endothelmarker in Violett (C) dargestellt. Die Zellkerne sind durch den Farbstoff DAPI blau gefärbt und dienen der Orientierung. Die Überlagerung der beiden Aufnahmen (B) zeigt, dass sich die QDQRs zum Teil in den Leberendothelzellen befinden, da sich die violette Färbung (Endothelmarker) und die Fluoreszenz der QDQRs an einigen Stellen überschneiden. Zusätzlich ist in diesen Bildern erneut die Aufnahme der QDQRs**-17a** in Kupffer-Zellen zu erkennen (größere Strukturen in Rot). In Figur 8 D - F sind die mikroskopischen Aufnahmen eines Leberschnittes einer Maus gezeigt, der QDQRs**-17b** injiziert wurden. Auch hier ist in der Überlagerung (E) der Fluoreszenz der QDQRs und des Endothelmarkers die Überschneidung von roten und violetten Bereichen zu sehen. Es kann demnach von einer Aufnahme der QDQRs in LSECs ausgegangen werden.

Figur 8 zeigt mikroskopische Aufnahmen eines Leberausschnittes nach Injektion von QDQRs-17a (A - C) bzw. QDQRs-17b (D - F). Die Fluoreszenz der QDQRs ist in Rot dargestellt (A und D), das Leberendothel ist mit CD31 markiert und in Violett dargestellt (C und F) und die Zellkerne sind mit DAPI gefärbt (blau). Aufnahmen B und E zeigen die Überlagerung von QDQRs und Endothelmarker.

### Beispiel 24

### Organverteilung von QDQRs-17a und QDQRs-17b ex vivo

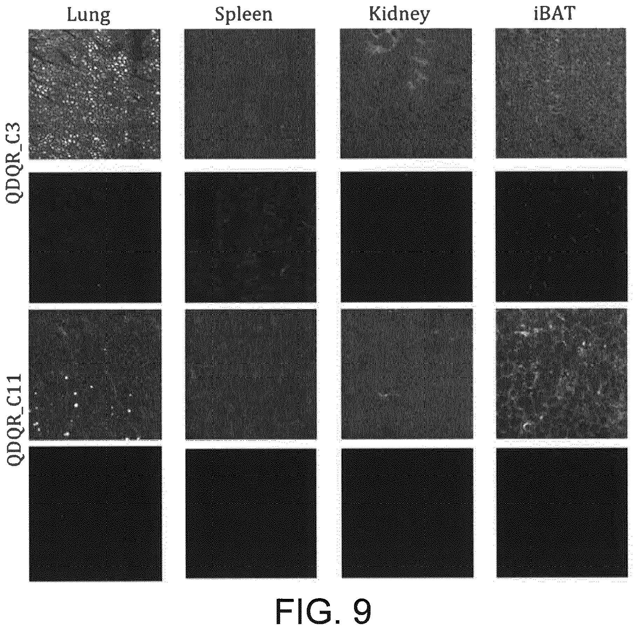

Nachdem das Verhalten der QDQRs in der Leber mittels Intravital-Mikroskopie analysiert wurde und die Aufnahme der Nanopartikel in LSECs bzw. Kupffer-Zellen durch weitere Experimente belegt werden konnte, wurde die Organverteilung der QDQRs ex *vivo* untersucht. Dazu wurden nach dem Perfundieren der Mäuse Lunge, Milz, Nieren und interscapulares braunes Fettgewebe (i-BAT) seziert und ebenfalls mikroskopisch analysiert. Figur 9 zeigt mikroskopische Aufnahmen von QDQRs-**17a und** QDQRs-**17b** in verschiedenen Geweben von Lunge, Milz, Niere und interscapularem braunem Fettgewebe. QDQRs-**17b** zeigten keine Aufnahme in den untersuchten Geweben. Im Gegensatz dazu wurden QDQRS-**17a** sowohl in der Lunge als auch in der Milz aufgenommen, wobei das Signal der Nanopartikel in diesen Organen weitaus geringer im Vergleich zur Leber war. Die Beobachtungen aus den *in vivo* Experimenten decken sich folglich mit den Ergebnissen aus den *in vitro* Untersuchungen, in denen QDQRs-**17a** eine unspezifische Aufnahme in A549-Zellen zeigten, wohingegen QDQRs-**17b** nur unter drastischen Bedingungen (lange Inkubationszeiten und ohne Zusatz von FCS) aufgenommen wurden.

Für QDQRs-**17b** eröffnen sich daher interessante therapeutische Perspektiven, da sie vornehmlich nur in LSECs aber nicht in Kupffer-Zellen aufgenommen werden. Aus der Literatur ist bekannt, dass LSECs eine große Rolle in der peripheren Immunregulation durch regulatorische T-Zellen spielen.¹¹,

Nanopartikel, die spezifisch von LSECs aufgenommen werden, können beispielsweise mit bestimmten Autoantigen-Peptiden gekoppelt werden. Durch nanopartikelvermitteltes Targeting können LSECs diese Autoantigen-Peptide prozessieren und präsentieren. Carambia *et al.* zeigten, dass dies zu einer Induktion regulatorischer T-Zellen (Tregs) führt, die das Autoantigen erkennen.¹ Tregs wirken als Immunmodulatoren. Sie können Immunantworten dämpfen und so zur Toleranz gegenüber Antigenen beitragen.

In einem Mausmodell von Multipler Sklerose (MS) wurden Nanopartikel injiziert, an denen Peptide des Myelin-Basischen-Proteins gekoppelt waren. Dies führte zu einer Induktion spezifischer Tregs, die das Fortschreiten der MS unterdrücken konnten.¹ Dieser Effekt war dabei allein von der Induktion der Tregs durch die LSECs abhängig. Somit könnten QDQRs-**17b** eine Möglichkeit darstellen, Substanzen spezifisch in LSECs zu transportieren, um immunoregulatorische Effekte zu vermitteln. Zu diesen Substanzen können Peptide oder auch andere biologisch-wirksame Moleküle wie Inhibitoren von intrazellulären Signalwegen gehören, die eine immunmodulatorische Wirkung aufweisen.

### Referenzbeispiel 25

### Synthese von 2-((11-bromundecyl)oxy)tetrahydro-2H-pyran

Nach Yang, C.^{[12]} wurde 11-Bromundecan-1-ol (10.67 g, 41.6 mmol, 1 eq.) in MTBE (94 mL, 0.80 mol, 19.2 eq.) gelöst und mit DHP (10.27 mL, 117 mmol, 2.81 eq.) versetzt. Nach kurzem Rühren bei Raumtemperatur (RT) wurde p-TsOH (53 mg, 308 µmol, 0.007 eq) als Katalysator langsam zugegeben und für vier Stunden bei RT gerührt.

Dünnschichtchromatographie (DC) mit Hex:EtOAc 19:1 zeigte eine Umsetzung. In einer Mikroaufarbeitung (2 mL) wurde mit Na₂CO₃-Lsg. (4 mL) gewaschen, um *p*-TsOH zu neutralisieren, über Na₂SO₄ getrocknet und zur Trockene eingeengt. Das ¹H-NMR-Spektrum war im Einklang mit der Zielstruktur. Daher wurde der Ansatz vollständig aufgearbeitet, durch dreimaliges Waschen mit Na₂CO₃-Lsg., einmaligem Waschen mit H₂O, Trocknung über Na₂SO₄ und Einengung zur Trockene. Das Produkt wurde als farbloses ÖI in 94 % Ausbeute erhalten. Die spektroskopischen Daten entsprachen der Literatur.^{[13]}

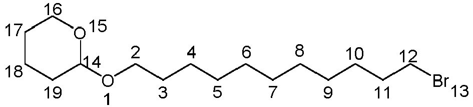

¹H-NMR (400 MHz, CDCl₃, 27 °C, TMS): δ [ppm] = 4.57 - 4.49 (m, 1 H, H-14); 3.86 - 3.76 (m, 1 H, H-16); 3.72 - 3.62 (dt, *J³*= 9.6, 6.9 Hz, 1 H, H-16); 3.49 - 3.40 (m, 1 H, H-2); 3.38 - 3.27 (m, 1 H, H-2; 2 H, H-12); 1.86 - 1.72 (m, 1 H, H-18; 2 H, H-11); 1.71 - 1.60 (m, 1 H, H-19); 1.59 - 1.42 (m, 2 H, H-17; 1 H, H-18; 1 H, H-19; 2 H, H-3); 1.41 - 1.16 (m, 14 H, H-4-H-10).

¹³C-NMR (400 MHz, CDCl₃, 27 °C, TMS): δ [ppm] = 98.78 (C-14); 67.62 (C-2); 62.25 (C-16); 33.86 (C-12); 32.86 (C-11); 30.81 (C-19); 29.78 (C-3); 29.54 (C-7); 29.48 (C-6, C-8); 29.43 (C-5); 28.77 (C-9); 28.19 (C-10); 26.26 (C-17); 25.57 (C-4); 19.70 (C-18).

ESI-MS: (m/z) ber. für C₁₆H₃₁BrO₂ (M+H)⁺ 334.1507, gef. 339.1862.

IR (neat): *ṽ* [cm⁻¹] = 2923 (C-H); 2853 (O-CH-O); 1077 (CH-O-CH).

### Referenzbeispiel 26

### Synthese von Diethyl(11-hydroxyundecyl)phosphonat

Nach Yang, C.^{[67]} wurde 2-((11-bromundecyl)oxy)tetrahydro-2H-pyran (5.51 g, 14.9 mmol, 1eq.) in P(OEt)₃ (6 mL, 34.3 mmol, 2.3 eq.) gelöst, bei 135°C für zehn Stunden zur Reaktion gebracht, welche per DC in Hex:EtOAc 1:1 überwacht wurde und anschließend weitere zehn Stunden bei RT gerührt. Nach der Entfernung von P(OEt)₃an einer Ölpumpe zeigte das ¹H-NMR-Spektrum einen sehr geringen Umsatz. Daher wurde eine kleine Menge der Reaktionslösung (1 mL) mit P(OEt)₃ (1.2 mL) bei 165 °C in der Mikrowelle [MW] für eine Stunde unter einem Druck von 4.4 bar gerührt. Die Reaktionskontrolle per NMR wiederum zeigte, dass die Reaktion stattgefunden hatte. Sodann wurde der komplette Ansatz (4.7 g) nach Zugabe von P(OEt)₃ (5.2 mL) für 90 Minuten bei 165 °C unter einem Druck von 2.7 bar zur Reaktion gebracht.

Die Reaktionslösung wurde bei 70 °C unter Vakuum von P(OEt)₃ befreit und durch Säulenchromatographie (100 mL Flash-Säule [FS]; 50 g Kieselgel [KG]) gereinigt.

[100 mL Hex, 200 mL Hex:EtOAc 4:1,300 mL Hex:EtOAc 7:3,300 mL Hex:EtOAc 3:2, 500 mL 1:1 Hex:EtOAc, 200 mL 1:4 Hex:EtOAc, 100 mL EtOAc]

Während der Säulenchromatographie wurden die Fraktionen per DC überwacht. Vor Vereinigung der organischen Phasen mit vermutetem Produkt wurden diese getrennt per NMR überprüft. Das ¹H-NMR-Spektrum zeigte, dass ein 1.2:1 Produktgemisch aus gewünschtem Produkt und **Diethyl(ethylphosphonat)** vorlag. Die spektroskopischen Daten der beiden Substanzen entsprachen der Literatur.^{[14,15]}

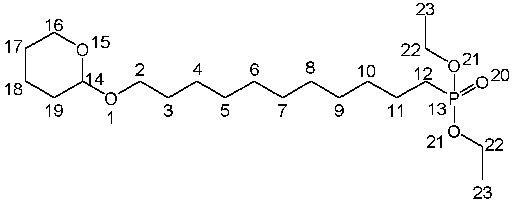

¹H-NMR (500 MHz, CDCl3, 27 °C, TMS): δ [ppm] = 4.61 - 4.52 (t, *J³*= 3.6 Hz, 1 H, H-14); 4.20 - 3.98 (m, 4 H, H-22); 3.90 - 3.80 (m, 1 H, H-16); 3.77 - 3.66 (m, 1 H, H-16); 3.54 - 3.43 (m, 1 H, H-2); 3.43 - 3.30 (m, 1 H, H-2); 1.90 - 1.64 (m, 1 H, H-18; 2 H, H-11; 1 H, H-19); 1.63 - 1.44 (m, 2 H, H-17; 1 H, H-18; 1 H, H-19; 2 H, H-3); 1.40 - 1.24 (m, 6 H, H-23; 14 H, H-3 - H-1 0).

¹³C-NMR (500 MHz, CDCl3, 27 °C, TMS): δ [ppm] = 98.97 (C-14); 67.81 (C-2); 62.48 (C-16); 61.51 (C-22); 30.81 (C-12); 29.87 (C-11); 29.66 (C-19); 29.59 (C-3); 29.48 (C-6, C-8); 29.21 (C-7); 26.36 (C-5); 25.63 (C-9); 22.51 (C-10); 19.84 (C-17); 19.51 (C-4); 18.38 (C-18); 16.59 (C-23).

ESI-MS: (m/z) ber. für C₂₀H₄₁O₅P (M+H)⁺ 392.2692, gef. 309.2229 (Fragment nach der Abspaltung von DHP).

IR (neat): *ṽ* [cm⁻¹] = 2981 (C-H); 2929 (C-H); 2856 (O-CH-O); 1247 (R(R'O)₂P=O); 1077 (CH-O-CH); 957 (P-O-C₂H₅).

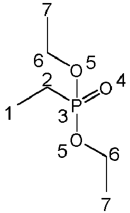

### Diethyl(ethylphosphonat)

¹H-NMR (500 MHz, CDCl₃, 27 °C, TMS): δ [ppm] = 4.17 - 3.97 (m, 4 H, H-6); 1.80 - 1.63 (dq, *J³*= 18.3, 7.7 Hz, 2 H, H-2); 1.34- 1.24 (t, *J³*= 7.1 Hz, 6 H, H-7); 1.20 - 1.06 (dt, *J³*= 20.0, 7.7 Hz, 3 H, H-1).

¹³C-NMR (500 MHz, CDCl₃, 27 °C, TMS): δ [ppm] = 61.51 (C-6); 19.51 (C-2); 16.61 (C-7), 6.71 (C-1).

ESI-MS: (m/z) ber. für C₆H₁₅O₃P (M+H)⁺ 166.0759, gef. 160.6168.

IR (neat): *ṽ* [cm⁻¹] = 2982 (C-H); 2942 (C-H); 2233 (R(R'O)₂P=O); 1247 (R(R'O)₂P=O).

Zur Entschützung wurde nach Azzouz, R.^{[16]} Amberlyst-15 H genutzt. Dieser wurde durch Spülen mit MeOH x2, H₂O x2, HCl 1M x1, HCl halbkonz. x1, H₂O [bis pH=7] x4 und MeOH x2 {1x = 20 mL} aktiviert.

Das Produktgemisch wurde zwei Stunden bei 45 °C in MeOH (10 mL) gerührt. Das ¹H-NMR-Spektrum war im Einklang mit der Zielstruktur. Das gewünschte Produkt wurde als trübes farbloses Öl in 83 % Ausbeute erhalten. Die spektroskopischen Daten entsprachen der Literatur.^{[64]}

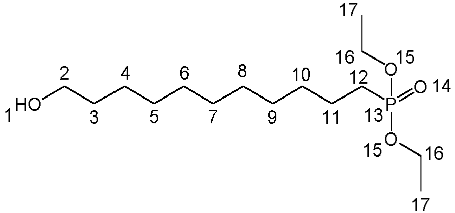

¹H-NMR (400 MHz, DMSO-d6, 27 °C, TMS): δ [ppm] = 4.59 - 4.02 (s br, 1H, H-1); 4.08 - 3.88 (m, 4 H, H-16); 3.48 - 3.37 (t, *J³* = *6.5* Hz, 2 H, H-2); 1.76 - 1.61 (m, 2 H, H-12); 1.51 - 1.18 (m, 6 H, H-17; 16 H, H-3-H-10).

¹³C-NMR (600 MHz, DMSO, 27 °C, TMS): δ [ppm] = 60.77 - 60.66 (C-2, C-16); 32.54 (C-3); 29.82 (C-11); 29.66 (C-7); 28.81 (C-6, C-8); 28.51 (C-5, C-9); 25.49 (C-10); 25.16 (C-4); 16.28 (C-17). C-12.

^{31p-}NMR (400 MHz, DMSO, 27 °C, H₃P0₄): δ [ppm] = 31.94 (P-13).

ESI-MS: (m/z) ber. für C₁₅H₃₃O₄P (M+H)⁺ 308.2116, gef. 309.2199.

IR (neat): *ṽ* [cm⁻¹] = 3418 (O-H); 2981 (C-H); 2926 (C-H); 2854 (-CH₂-); 1220 (R(R'O)₂P=O); 1022 (C-O); 955 (P-O-C₂H₅).

### Referenzbeispiel 27

### Synthese von Diethyl(11-(trityloxy)undecyl)phosphonat

Nach Rele, S.^{[17]} wurde eine Lösung aus 11-Bromundecan-1-ol (1.283 g, 5.01 mmol, 1eq.) in DCM (12 mL, 188 mmol, 37.5 eq.) in einem Eisbad gekühlt, mit NEt₃ (837 µL, 6.01 mmol, 1.2 eq.) versetzt, bevor eine Lösung aus Tritylchlorid (1.577 g, 5.54 mmol, 1.1 eq.) in DCM (3 mL, 47 mmol, 9.4 eq.) langsam zugetropft wurde. Nach 30 Minuten wurde das Eisbad entfernt. Nach vier Stunden war die Reaktion laut DC in Hex:EtOAc 9:1 für beendet erklärt.

Das bei der Reaktion entstandene Triethylaminhydrochlorid [NEts HCl] wurde abfiltriert.

Das Rohgemisch wurde säulenchromatisch mit Hex:EtOAc 19:1 gereinigt. Die vereinigten organischen Phasen wurden zur Trockene eingeengt.

Das ¹H-NMR-Spektrum war im Einklang mit der Zielstruktur. Das gewünschte Produkt wurde als trübes farbloses Öl in 70 % Ausbeute erhalten.

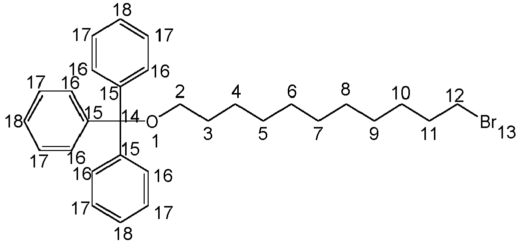

¹H-NMR (600 MHz, DMSO, 27 °C, TMS): δ [ppm] = 7.40 - 7.34 (d, *J*³= 8.0 Hz, 6 H, H-16); 7.34 - 7.29 (t, *J³*= 7.7 Hz, 6 H, H-17); 7.27 - 7.21 (t, *J³=* 7.2 Hz, 3 H, H-18); 3.52 - 3.45 (t, *J³*= *6.7* Hz, 2 H, H-12); 2.99 - 2.90 (t, *J³=* 6.5 Hz, 2 H, H-2); 1.81 - 1.71 (p, *J³*= *6.8* Hz, 2 H, H-11); 1.57-1.48 (p, *J³*= 6.6 Hz, 2 H, H-3); 1.41 -1.16 (m, 14 H, H-4 - H-10).

¹³C-NMR (600 MHz, DMSO, 27 °C, TMS): δ [ppm] = 144.05 (C-15); 128.12 (C-16); 127.75 (C-17); 126.84 (C-18); 85.71 (C-14); 62.83 (C-2); 35.07 (C-12); 32.21 (C-11); 29.26 (C-3); 28.81 (C-7); 28.77 (C-6, C-8); 28.65 (C-5); 28.06 (C-9); 27.47 (C-10); 25.62 (C-4).

ESI-MS: (m/z) ber. für C₃₀H₃₇BrO (M+H)⁺ 492.2028, gef. 243.1187 (Tritylgruppe) (ber. 243.1174).

IR (neat): *ṽ* [cm⁻¹] = 3059 (C=C-H); 2927 (C-H); 2854 (-CH₂-); 1072 (C-O); 705 (R-Car).

### Referenzbeispiel 28

### Synthese von Diethyl(11-bromundecyl)phosphonat

Nach Baughman, T. W.^{[18]} wurde das vorstehend erhaltene Diethyl(11-hydroxyunde-cyl)phosphonat (1.01 g, 3.28 mmol, 1 eq.) in DCM (10 mL, 157 mmol, 47.9 eq.) im Eisbad gelöst, mit PPh₃ (945 mg, 3.6 mmol, 1.1 eq.) versetzt und langsam mit CBr₄ (1.195 g, 3.6 mmol, 1.1 eq.) unter Rühren versetzt. Nach 30 Minuten wurde das Eisbad entfernt und das Gemisch für zweieinhalb Stunden weitergerührt. Die Reaktionslösung wurde nach einer Mikroaufarbeitung (80 µL) per NMR kontrolliert, wonach ein 1:1 Gemisch aus Edukt und Produkt in der Reaktionslösung vorlag. Während die Reaktion bei 40 °C für zwei Stunden weitergeführt wurde, konnte per ESI-MS das Produkt anhand des charakteristischen Isotopenmusters nachgewiesen werden. Aufgrund der geringen Löslichkeit des Produktes in Hex wurde der nach Eindampfen der Reaktionslösung erhaltene Rückstand mit heißem Hex (5x 100 mL) trituriert sowie der Überstand zur Trockene eingeengt. Säulenchromatographie (100 mL FS, 50g KG) [400 mL Hex:EtOAc 4:1, 200 mL Hex:EtOAc 7:3, 200 mL Hex:EtOAc 3:2, 500 mL Hex:EtOAc 1:1] erbrachte das gewünschte Produkt als gelbliches Öl in 58 % Ausbeute.

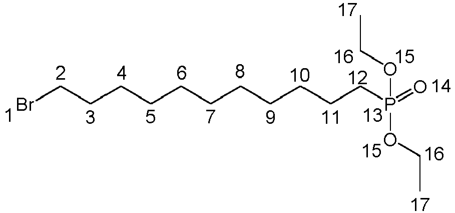

¹H-NMR (600 MHz, CDCl3, 27 °C, TMS): δ [ppm] = 4.13 - 4.02 (m, 4 H, H-16); 3.41 - 3.37 (t, *J³*= 6.9 Hz, 2 H, H-2); 1.87 - 1.81 (dt, *J³*= 14.6, 6.9 Hz, 2 H, H-3); 1.74-1.66 (m, 2 H, H-12); 1.62 - 1.53 (m, 2 H, H-11); 1.44 - 1.21 (m, 6 H, H-17; 14 H, H-4 - H-10).

¹³C-NMR (600 MHz, CDCl3, 27 °C, TMS): δ [ppm] = 61.52 (C-16); 34.14 (C-2); 32.95 (C-3); 30.77 (C-12), 30.66 (C-11); 29.50 (C-7, C-8); 29.19 (C-6); 28.86 (C-5); 28.28 (C-9); 26.29 (C-10); 25.36 (C-4); 16.61 (C-17).

^{31p-}NMR (600 MHz, CDCl3, 27 °C, H₃P0₄): δ [ppm] = 32.65 (P-13).

ESI-MS: (m/z) ber. für C₁₅H₃₂BrO₃P (M+H)⁺ 370.1272, gef. 371.1270.

IR (neat): *ṽ* [cm⁻¹] = 2926 (C-H); 2854 (-CH₂-); 1244 (R(R'O)₂P=O); 1060 (C-O); 1029 (C-O); 960 (P-O-C₂H₅).

### Referenzbeispiel 29

Synthese von Diethyl(11-bromundecyl)phosphonat aus 1,11-Dibromundecan Nach Tisato, F.^{[19]} wurde 1,11-Dibromundecan (5.24 mL, 16.4 mmol, 4 eq.) in P(OEt)₃ (716 µL, 4.09 mmol, 1 eq.) gelöst und bei 200 °C unter Rückfluss für zwei Stunden zur Reaktion gebracht. Das freiwerdende Ethylbromid wurde durch ein mit Aktivkohle beladenes Trockenrohr zurückgehalten. Die Reaktionskontrolle erfolgte per DC und per NMR.

Zur Aufreinigung wurde das leichter flüchtige Edukt bei 150 °C im Vakuum (0.1 mbar) mit 15 cm Vigreux-Kolonne über zwei Stunden abdestilliert. Die ¹H-NMR-Spektren zeigten 9.6 % Produkt im Destillat sowie 14 % Edukt im Sumpf an. Das übrige Reaktionsgemisch wurde säulenchromatographisch (100 mL FS; 50 g KG) mit einem Stufengradienten von 10 % mit je 200 mL [Hex/Hex:EtOAc 3:7] gereinigt.

Das ¹H-NMR-Spektrum war im Einklang mit der Zielstruktur. Das Produkt wurde als trübes Öl in 90 % Ausbeute erhalten.

### Referenzbeispiel 30

### Synthese von Diethyl(11-(dimethylamino)undecyl)phosphonat

Diethyl(11 -bromundecyl)phosphonat (440 mg, 1.185 mmol, 1 eq.) wurde in einer 5.6 M Lösung aus Dimethylamin (NMe₂) in absolutem Ethanol [EtOH_{abs}] (2.16 mL, 11.85 mmol, 10 eq.) gelöst und bei 60 °C für in MW zwei Stunden gerührt. Die Reaktion wurde per DC in 98:2 DCM:MeOH überwacht.

Die Reaktionslösung wurde im Ölpumpenvakuum von Lösungsmittel und NMe₂ befreit, in MTBE (10 mL) suspendiert und mit NaOH 1 M (5 mL) extrahiert, um das gebildete Hydrobromid, in die freie Base zu überführen. Die organische Phase wurde mit Na₂SO₄ getrocknet und zur Trockene eingeengt. Das Produkt fiel als farbloses Öl in 91 % Ausbeute an.

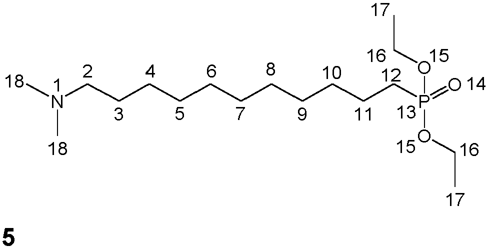

¹H-NMR (500 MHz, CDCl3, 27 °C, TMS): δ [ppm] = 4.16 - 4.00 (m, 4 H, H-16); 2.34 - 2.18 (m, 2 H, H-2; 6 H, H-18); 1.74 - 1.66 (m, 2 H, H-12); 1.64 - 1.51 (m, 2 H, H-3); 1.51 - 1.41 (m, 2 H, H-11); 1.39- 1.16 (m, 6 H, H-17; 14 H, H-4-H-10).

¹³C-NMR (500 MHz, CDCl₃, 27 °C, TMS): δ [ppm] = 61.49 (C-16); 59.96 (C-2); 45.45 (C-18); 30.81 (C-12); 30.68 (C-11); 29.65 (C-7, C-8); 29.48 (C-3); 29.21 (C-6); 27.65 (C-5); 27.57 (C-9); 26.39 (C-10); 25.27 (C-4); 16.62 (C-17).

ESI-MS: (m/z) ber. für C₁₇H₃₈NO₃P (M+H)⁺ 335.2589, gef. 336.2672.

IR (neat): *ṽ* [cm⁻¹] = 3423 (N-H); 2981 (C-H); 2927 (C-H); 2855 (-CH₂-); 1231 (R(R'O)₂P=O); 1065 (C-O); 1027 (C-O); 963 (P-O-C₂H₅).

### Referenzbeispiel 31

### Synthese von 3-((11-(Diethoxyphosphoryl)undecyl)dimethylammonio)-propan-1-sulfonat

Diethyl(11-(dimethylamino)undecyl)phosphonat (300 mg, 894 µmol, 1 eq.) wurde in trockenem CHCl3 (6 mL 83.8 eq.) gelöst, mit 1,3-Propansulton (83 µL, 921 µmol, 1.03 eq.) versetzt und bei 100 °C in einem MW-Ofen für zwei Stunden gerührt.

Das Produkt wurde mit 6:1 MTBE:EtOAc (40 mL) gefällt, durch Zentrifugieren abgetrennt, in reinem EtOAc (40 mL) suspendiert, erneut zentrifugiert sowie in H₂O (10 mL) gelöst. Lyophilisierung erbrachte einen hygroskopischen Feststoff in 59 % Ausbeute.

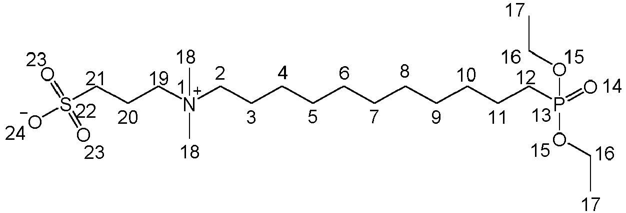

¹H-NMR (400 MHz, D₂0, 27 °C, TMS): δ [ppm] = 4.22 - 4.11 (pd, *J³*= 7.1, 2.3 Hz, 4 H, H-16);

3.53 - 3.46 (m, 2H, H-21); 3.39 - 3.31 (m, 2 H, H-19); 3.17 - 3.09 (s, 6 H, H-18); 3.05 -2.99 (t, *J³=* 7.2 Hz, 2 H, H-2), 2.30 - 2.20 (m, 2 H, H-20); 1.98 - 1.87 (m, 2 H, H-12); 1.85 - 1.75 (m, 2 H, H-3); 1.68 - 1.55 (m, 2 H, H-11); 1.49 - 1.30 (m, 6 H, H-17; 14 H, H-4 - H-10).

¹³C-NMR (400 MHz, D₂0, 27 °C, TMS): δ [ppm] = 64.37 (C-2); 63.10 (C-16); 62.07 (C-19); 50.58 (C-21); 47.31 (C-18); 29.50 (C-12); 28.56 (C-11); 28.47 (C-7, C-8); 28.17 (C-20); 25.49 (C-3); 24.35 (C-6); 23.00 (C-5); 21.83 (C-9); 21.42 (C-10); 18.17 (C-4); 15.64 (C-17).

³¹P-NMR (400 MHz, D₂0, 27 °C, H₃P0₄): δ [ppm] = 31.94 (P-13).

ESI-MS: (m/z) ber. für C20H44N06PS (M+H)+ 457.2627, gef. 458.2713.

IR (neat): *ṽ* [cm⁻¹] = 3313 (R₂R'R"N⁺); 2504 (R₂R'R"N⁺); 1636 (R(R'O)₂P=O); 1457 (-O-CH₂-); 1045 (S=O); 589 (R(R'O)₂P=O); 589 (C-C).

### Referenzbeispiel 32

### Synthese von 3-(Dimethyl(11-phosphonoundecyl)ammonio)propan-1-sulfonat

Eine 23 mM Lösung von 3-((11-(Diethoxyphosphoryl)undecyl)dimethylammonio)-propan-1-sulfonat (104 mg, 227 µmol) in Millipore-H₂O wurde unter Mikrowellenheizung bei 250 °C und einem Druck von ca. 40 bar für eine Stunde hydrolysiert. Durch Lyophilisierung wurde ein weißer Feststoff erhalten, der laut ¹H-NMR-Spektrum dem gewünchten Produkt in 94 % Ausbeute entsprach.

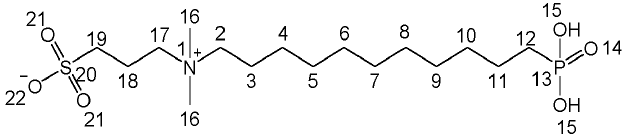

¹H-NMR (400 MHz, D₂0, 27 °C, TMS): δ [ppm] = 3.53 - 3.46 (m, 2H, H-19); 3.39 - 3.31 (m, 2 H, H-17); 3.17 - 3.09 (s, 6 H, H-16); 3.03 - 2.97 (t, *J³*= 7.2 Hz, 2 H, H-2), 2.34 - 2.15 (m, 2 H, H-20); 1.88 - 1.70 (dt, *J³*= 17.2, 8.2 Hz, 4 H, H-3, H-12); 1.67 - 1.51 (m, 2 H, H-11); 1.48-1.27 (m, 14 H, H-4-H-10).

¹³C-NMR (400 MHz, D₂0, 27 °C, TMS): δ [ppm] = 64.39 (C-2); 62.06 (C-17); 50.55 (C-19); 47.30 (C-16); 29.79 (C-12); 29.63 (C-11); 28.48 (C-7, C-8); 28.10 (C-18); 26.96 (C-3); 25.64 (C-6); 25.41 (C-5); 22.08 (C-9); 21.78 (C-10); 18.15 (C-4).

³¹P-NMR (400 MHz, D₂0, 27 °C, H₃P0₄): δ [ppm] = 32.14 (P-13).

ESI-MS: (m/z) ber. für C₁₆H₃₆NO₆PS (M+H)⁺ ber. 401.2001, gef. 402.2077.

IR (neat): *ṽ* [cm⁻¹] = 2915 (C-H); 2849 (-CH₂-); 2291 (R₂R'R"N⁺); 1653 (R(HO)₂P=O); 1467 (C-H); 1219 (-S03-)- 7 1149 (-S03-)- 7 1034 (C-N); 974 (C-C); 940 (P-O); 768 (C-C); 730 (C-C); 521 (C-C); 491 (C-C); 422 (R(HO)₂P=O).

### Referenzbeispiel 32

### Synthese von (11-(Dimethylamino)undecyl)phosphonsäure

Eine 23 mM Lösung von Diethyl(11-(dimethylamino)undecyl)phosphonat (100 mg, 298 µmol) in Millipore-H₂O wurde unter Mikrowellenheizung bei 250 °C und einem Druck von ca. 40 bar^{[53]} für eine Stunde zur Reaktion gebracht. Durch Lyophilisierung wurde ein weißer Feststoff erhalten, der laut ¹H-NMR-Spektrum das gewünschte Produkt in 94 % Ausbeute entsprach.

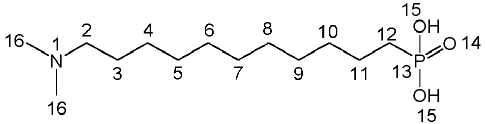

¹H-NMR (400 MHz, D₂O, 27 °C, TMS): δ [ppm] = 3.43 - 3.35 (q, *J³*= 7.3 Hz, 2 H, H-2); 3.32 - 3.24 (m, 2 H, H-15); 3.09 - 3.00 (s, 6 H, H-16); 1.84 - 1.69 (m, 2 H, H-12); 1.69 - 1.48 (m, 4 H, H-3, H-11); 1.46-1.26 (m, 14 H, H-4-H-10).

¹³C-NMR (400 MHz, D₂0, 27 °C, TMS): δ [ppm] = 60.31 (C-2); 42.50 (C-16); 38.93 (C-12); 30.51 (C-11); 30.34 (C-8); 28.93 (C-7); 28.52 (C-3); 28.34 (C-6); 28.08 (C-5); 27.62 (C-9); 26.74 (C-10); 25.43 (C-4).

³¹P-NMR (400 MHz, D₂0, 27 °C, H₃P0₄): δ [ppm] = 27.74 (P-13).

ESI-MS: (m/z) ber. für C₁₃H₃₀NO₃P ((M+H)+ 279.1963, gef. 280.2091.

IR (neat): *ṽ* [cm⁻¹] = 3399 (N-H); 2919 (C-H); 2850 (-CH₂-); 2345 (R(HO)₂P=O); 1576 (C-N); 1416 (C-H); 1220 (R(R'O)₂P=O); 1158 (C-N), 1035 (S=O), 892 (P-O), 780 (C-C), 470 (C-C).

### Referenzen:

[1]Carambia, A.; Freund, B.; Schwinge, D.; Bruns, O. T.; Salmen, S. C.; Ittrich, H.; Reimer, R.; Heine, M.; Huber, S.; Waurisch, C.; et al. Nanoparticle-Based Autoantigen Delivery to Treg-Inducing Liver Sinusoidal Endothelial Cells Enables Control of Autoimmunity in Mice. J. Hepatol. 2015, 62, 1349-1356.
[2]Carambia, A.; Freund, B.; Schwinge, D.; Heine, M.; Laschtowitz, A.; Huber, S.; Wraith, D. C.; Korn, T.; Schramm, C.; Lohse, A. W.; etal. TGF-β-Dependent Induction of CD4+CD25+Foxp3+ Tregs by Liver Sinusoidal Endothelial Cells. J. Hepatol. 2014, 61, 594-599.
[3] Longmire, M. R.; Ogawa, M.; Choyke, P. L.; Kobayashi, H. Biologically Optimized Nanosized Molecules and Particles: More Than Just Size. Bioconjug. Chem. 2011, 22, 993-1000.
[4] Petros, R. A.; DeSimone, J. M. Strategies in the Design of Nanoparticles for Therapeutic Applications. Nat. Rev. Drug Discov. 2010, 9, 615-627.
[5] Schipper, M. L.; Iyer, G.; Koh, A. L.; Cheng, Z.; Ebenstein, Y.; Aharoni, A.; Keren, S.; Bentolila, L. A.; Li, J.; Rao, J.; et al. Particle Size, Surface Coating, and PEGylation Influence the Biodistribution of Quantum Dots in Living Mice. Small 2009, 5, 126-134.
[6] Fischer, H. C.; Liu, L.; Pang, K. S.; Chan, W. C. W. Pharmacokinetics of Nanoscale Quantum Dots: In Vivo Distribution, Sequestration, and Clearance in the Rat. Adv. Funct. Mater. 2006, 16, 1299-1305.
[7] Ilium, L.; Davis, S. S.; Wilson, C. G.; Thomas, N. W.; Frier, M.; Hardy, J. G. Blood Clearance and Organ Deposition of Intravenously Administered Colloidal Particles. The Effects of Particle Size, Nature and Shape. Int. J. Pharm. 1982, 12, 135-146.
[8] Carambia, A.; Freund, B.; Schwinge, D.; Bruns, O. T.; Salmen, S. C.; Ittrich, H.; Reimer, R.; Heine, M.; Huber, S.; Waurisch, C.; et al. Nanoparticle-Based Autoantigen Delivery to Treg-Inducing Liver Sinusoidal Endothelial Cells Enables Control of Autoimmunity in Mice. J. Hepatol. 2015, 62, 1349-1356.
[9] Sørensen, K. K.; McCourt, P.; Berg, T.; Crossley, C.; Le Couteur, D.; Wake, K.; Smedsrød, B. The Scavenger Endothelial Cell - A New Player in Homeostasis and Immunity. Am. J. Physiol. Regul. Integr. Comp. Physiol. 2012, 303, R1217-R1230.
[10]Kamimoto, M.; Rung-Ruangkijkrai, T.; Iwanaga, T. Uptake Ability of Hepatic Sinusoidal Endothelial Cells and Enhancement by Lipopolysaccharide. Biomed. Res. 2005, 26, 99-107.
[11] Carambia, A.; Freund, B.; Schwinge, D.; Heine, M.; Laschtowitz, A.; Huber, S.; Wraith, D. C.; Korn, T.; Schramm, C.; Lohse, A. W.; et al. TGF-β-Dependent Induction of CD4+CD25+Foxp3+ Tregs by Liver Sinusoidal Endothelial Cells. J. Hepatol. 2014, 61, 594-599.
[12] Yang, C.; Liu, H.; Zhang, Y.; Zhigang, X.; Wang, Y.; Cao, B.; Wang, M. Biomacromolecules, 2016, 17, 1673-1683.
[13] Perez-Balado, C.; Nebbioso, A.; Rodriguez-Graña, P.; Minichiello, A.; Miceli, M.; Altucci, L.; de Lera, Á. R. J. Med. Chem., 2007, 50, 2497-2505.
[14] Minet, I.; Delhalle, J.; Hevesi, L.; Mekhalif, Z. J. Colloid Interface Sci., 2009, 332, 2, 317-326.
[15] Lai, C.; Xi, C.; Chen, W.; Hua, R. Tetrahedron, 2006, 62, 6295-6302.
[16] Azzouz, R.; Bischoff, L.; Fouquet, M.-H.; Marsais, F. Synlett, 2005, 2808.
[17] Rele, S.; Nayak S. K. Synthetic Communications, 2002, 32, 22, 3533-3540.
[18] Baughman, T. W.; Sworen, J. C.; Wagener, K. B. Tetrahedron, 2004, 60, 10943-10948.
[19] Nihon Medi-Physics Co., Ltd., 2007, Intermediate compound of technetium nitride complex for radiodiagnostic imaging Erfinder: Tisato, F.; Refosco, F.; Bolzati, C.; et al., JP, Patentschrift: PCT/JP2006/301260.

## Patentansprüche

1. Verwendung eines zwitterionischen Nanopartikels für eine biologische Anwendung, wobei die biologische Anwendung eine immunologische Anwendung ist oder umfasst, wobei das zwitterionische Nanopartikel wenigstens einen Nanopartikel und eine zwitterionische Hülle aufweist, die den Nanopartikel umschließt.

2. Verwendung eines zwitterionischen Nanopartikels nach Anspruch 1, **dadurch gekennzeichnet, dass** das Nanopartikel ein lumineszentes und/oder magnetisches und/oder plasmonisches Nanopartikel ist.

3. Verwendung eines zwitterionischen Nanopartikels nach Anspruch 1, **dadurch gekennzeichnet, dass** das Nanopartikel wenigstens ein Wirkstoffmolekül enthält.

4. Verwendung eines zwitterionischen Nanopartikels nach Anspruch 1 oder nach Anspruch 2, **dadurch gekennzeichnet, dass** das Nanopartikel ein Halbleiternanopartikel ist.

5. Verwendung eines zwitterionischen Nanopartikels nach Anspruch 4, **dadurch gekennzeichnet, dass** das Nanopartikel mit Schwermetallionen, wie Ag, Cu, Co oder Mn dotiert ist.

6. Verwendung eines zwitterionischen Nanopartikels nach Anspruch 1 oder nach Anspruch 2, **dadurch gekennzeichnet, dass** das Nanopartikel ausgewählt ist aus lanthanid-dotierten Nanopartikeln mit salzartigen Wirtsgittern.

7. Verwendung eines zwitterionischen Nanopartikels nach Anspruch 1, **dadurch gekennzeichnet, dass** das Nanopartikel ein metallisches Nanopartikel ist, oder aus einem Metalloxid besteht.

8. Verwendung eines zwitterionischen Nanopartikels nach einem der vorhergehenden Ansprüche, **dadurch gekennzeichnet, dass** der Aufbau des Nanopartikels durch die Formel M_{w}NₓA_{y}B_{z} beschrieben wird, wobei M und N unabhängig voneinander ausgewählt sind aus den Elementen der Gruppen 8, 9, 10, 11, 12, 13 oder 14 des Periodensystems der Elemente, z. B., jedoch nicht eingeschränkt auf, Fe, Co, Ni, Pt, Cu, Zn, Cd, Al, Ga, In, Ge, Sn or Pb, und A und B unabhängig voneinander ausgewählt sind aus den Elementen 10, 11, 13, 15 oder 16 des Periodensystems der Elemente, z. B., jedoch nicht eingeschränkt auf Pd, Pt, N, P, As, Sb, Ga, O, S, Se oder Te und wobei w, x, y und z unabhängig voneinander ganzzahlige Vielfache eines Werts zwischen 0 und 1 annehmen kann, unter der Maßgabe, dass die positive Gesamtladung der kationischen Elemente entgegengesetzt gleich der negativen Ladung der anionischen Elemente ist.

9. Verwendung eines zwitterionischen Nanopartikels nach einem der vorhergehenden Ansprüche, **dadurch gekennzeichnet, dass** das Nanopartikel wenigstens von einer Schale umgeben ist.

10. Verwendung eines zwitterionischen Nanopartikels nach einem der vorhergehenden Ansprüchen, **dadurch gekennzeichnet, dass** die zwitterionische Verbindung durch die Formel A-X-[L¹-Z¹-L²-Z²]ₙ beschrieben wird, wobei
1. A wahlweise eine Gruppe mit Affinität zur Oberfläche des Nanopartikels ist oder ein mindestens zweiwertiges Atom, das geeignet ist, eine kovalente Bindung zum Nanopartikel auszubilden;
2. L¹ und L² voneinander unabhängige Linkergruppen sind;
3. X wahlweise ein optionales Verzweigungselement dergestalt ist, dass X zusätzlich zu der Bindung an A mindestens eine weitere Bindung zu L¹ aufweist;
4. n die ganzen Zahlen zwischen 1 und der maximalen Valenz von X-1 umfasst;
5. Z¹ eine erste geladene oder ionisierbare Gruppe einschließt; und
6. Z² eine zweite geladene oder ionisierbare Gruppe unter der Maßgabe einschließt, dass, falls Z¹ oder Z² Ladungen tragen, diese entgegengesetzt sind.

11. Verwendung eines zwitterionischen Nanopartikels nach Anspruch 10, **dadurch gekennzeichnet, dass** A aus der Gruppe C, N, O, S, P oder Si gewählt ist, wobei mindestens eine Amino-, Mercapto-, Dithiocabamat-, Carboxy, Phosphat-, PhosphonatGruppe vorgesehen ist.

12. Verwendung eines zwitterionischen Nanopartikels nach Anspruch 11, **dadurch gekennzeichnet, dass** L¹ und L² voneinander unabhängige lineare oder verzweigte, wahlweise gesättigte oder ungesättigte Kohlenwasserstoffketten mit ein bis vierzig C-Atomen, die wahlweise Heteroatome aus der Gruppe N, O, P, Si und S enthalten.

13. Verwendung einer Zusammensetzung, die wenigstens ein zwitterionisches Nanopartikel nach einem der vorhergehenden Ansprüche in wässriger Lösung enthält.

14. Verwendung nach einem der vorgenannten Ansprüche, wobei die immunologische Anwendung eine Modulation einer Entzündung ist oder umfasst, insbesondere wobei die Modulation einer Entzündung eine Reduktion autoaggressiver Entzündungen ist oder umfasst.

15. Verwendung nach einem der vorgenannten Ansprüche, wobei die immunologische Anwendung eine Modulation einer Autoimmunerkrankung ist oder umfasst.

16. Verwendung nach einem der vorgenannten Ansprüche, wobei die immunologische Anwendung eine Modulation einer Allergie ist oder umfasst.

17. Verwendung nach einem der vorgenannten Ansprüche, wobei die immunologische Anwendung eine Unterdrückung von immunologischen Antworten gegen Biologicals ist oder umfasst.

18. Verwendung nach einem der vorgenannten Ansprüche, wobei die biologische Anwendung eine Modulation einer Differenzierung einer Zelle, insbesondere einer Leberzelle, beispielsweise einer Leberendothelzelle, ist oder umfasst.

## Claims

1. Use of a zwitterionic nanoparticle for a biological application, wherein the biological application is or comprises an immunological application, wherein the zwitterionic nanoparticle comprises at least one nanoparticle and a zwitterionic shell surrounding said nanoparticle.

2. The use of a zwitterionic nanoparticle according to claim 1, **characterized in that** the nanoparticle is a luminescent and/or magnetic and/or plasmonic nanoparticle.

3. The use of a zwitterionic nanoparticle according to claim 1, **characterized in that** the nanoparticle comprises at least one active substance molecule.

4. The use of a zwitterionic nanoparticle according to claim 1 or 2, **characterized in that** the nanoparticle is a semiconductor nanoparticle.

5. The use of a zwitterionic nanoparticle according to claim 4, **characterized in that** the nanoparticle is doped with heavy metal ions such as Ag, Cu, Co or Mn.

6. The use of a zwitterionic nanoparticle according to claim 1 or 2, **characterized in that** the nanoparticle is selected from lanthanide-doped nanoparticles with salt-like host lattices.

7. The use of a zwitterionic nanoparticle according to claim 1, **characterized in that** the nanoparticle is a metal nanoparticle or consists of a metal oxide.

8. The use of a zwitterionic nanoparticle according to any of the preceding claims, **characterized in that** the structure of the nanoparticle is described by the formula M_{w}NₓA_{y}B_{z}, wherein M and N are independently selected from the elements of the groups 8, 9, 10, 11, 12, 13 or 14 of the periodic table of the elements, for instance, but not limited to Fe, Co, Ni, Pt, Cu, Zn, Cd, Al, Ga, In, Ge, Sn or Pb, and A and B are independently selected from the elements 10, 11, 13, 15 or 16 of the periodic table of the elements, for instance, but not limited to Pd, Pt, N, P, As, Sb, Ga, O, S, Se or Te, and wherein w, x, y and z may independently be integer multiples of a value between 0 and 1, with the proviso that the total positive charge of the cationic elements is oppositely equal to the negative charge of the anionic elements.

9. The use of a zwitterionic nanoparticle according to any of the preceding claims, **characterized in that** the nanoparticle is surrounded by at least one shell.

10. The use of a zwitterionic nanoparticle according to any of the preceding claims, **characterized in that** the zwitterionic compound is described by the formula A-X-[L¹-Z¹-L²-Z²]ₙ, wherein
1. A is selectively a group with affinity to the surface of the nanoparticle or is an at least divalent atom capable of forming a covalent bond to the nanoparticle;
2. L¹ and L² are linker groups independent of each other;
3. X is selectively an optional branching element such that X has at least one further bond to L¹ in addition to the bond to A; and
4. n comprises the integer numbers between 1 and the maximum valence of X-1,
5. Z¹ includes a first charged or ionizable group; and
6. Z² includes a second charged or ionizable group under the proviso that, if Z¹ or Z² carry charges, said charges are opposite.

11. The use of a zwitterionic nanoparticle according to claim 10, **characterized in that** A is selected from the group consisting of C N, O, S, P or Si, at least one amino, mercapto, dithiocarbamate, carboxyl, phosphate or phosphonate group being provided.

12. The use of a zwitterionic nanoparticle according to claim 11, **characterized in that** L¹ and L² are mutually independent, linear or branched, selectively saturated or unsaturated hydrocarbon chains having one to forty C atoms, which optionally contain heteroatoms from the group consisting of N, O, P, Si and S.

13. The use of a composition comprising at least one zwitterionic nanoparticle according to any of the preceding claims in aqueous solution.

14. The use according to any of the preceding claims, wherein the immunological application is or comprises a modulation of an inflammation, in particular wherein the modulation of an inflammation is or comprises a reduction of auto-aggressive inflammations.

15. The use according to any of the preceding claims, wherein the immunological application is or comprises a modulation of an autoimmune disease.

16. The use according to any of the preceding claims, wherein the immunological application is or comprises a modulation of an allergy.

17. The use according to any of the preceding claims, wherein the immunological application is or comprises suppression of immunological responses against biologicals.

18. The use according to any of the preceding claims, wherein the biological application is or comprises modulation of a differentiation of a cell, in particular a liver cell, for example a liver endothelial cell.

## Revendications

1. Utilisation d'une nanoparticule zwitteronique pour une application biologique, l'application biologique étant ou comprenant une application immunologique, la nanoparticule zwitteronique comportant au moins une nanoparticule et une enveloppe zwitteronique entourant la nanoparticule.

2. Utilisation d'une nanoparticule zwitteronique selon la revendication 1, **caractérisée en ce que** la nanoparticule est une nanoparticule luminescente et/ou magnétique et/ou plasmonique.

3. Utilisation d'une nanoparticule zwitteronique selon la revendication 1, **caractérisée en ce que** la nanoparticule contient au moins une molécule de substance active.

4. Utilisation d'une nanoparticule zwitteronique selon la revendication 1 ou selon la revendication 2, **caractérisée en ce que** la nanoparticule est une nanoparticule semiconductrice.

5. Utilisation d'une nanoparticule zwitteronique selon la revendication 4, **caractérisée en ce que** la nanoparticule est dotée d'ions de métaux lourds, tels que Ag, Cu, Co ou Mn.

6. Utilisation d'une nanoparticule zwitteronique selon la revendication 1 ou selon la revendication 2, **caractérisée en ce que** la nanoparticule est sélectionnée dans le groupe des nanoparticules dotées de lanthanides avec des grilles hôtes de type sel.

7. Utilisation d'une nanoparticule zwitteronique selon la revendication 1, **caractérisée en ce que** la nanoparticule est une nanoparticule métallique ou se compose d'un oxyde métallique.

8. Utilisation d'une nanoparticule zwitteronique selon l'une quelconque des revendications précédentes, **caractérisée en ce que** la structure de la nanoparticule est décrite par la formule M_{w}NₓA_{y}B_{z}, M et N étant choisis indépendamment l'un de l'autre à partir des éléments des groupes 8, 9, 10, 11, 12, 13 ou 14 du système périodique des éléments, par exemple, sans toutefois s'y limiter Fe, Co, Ni, Pt, Cu, Zn, Cd, Al, Ga, In, Ge, Sn or Pb, et A et B étant choisis indépendamment l'un de l'autre à partir des éléments 10, 11, 13, 15 ou 16 du système périodique des éléments, par exemple, toutefois sans s'y limiter Pd, Pt, N, P, As, Sb, Ga, O, S, Se ou Te et w, x, y et z pouvant prendre indépendamment les uns des autres une valeur de multiple entier comprise entre 0 et 1, en prenant en compte le fait que la charge totale positive des éléments cationiques est égale à la charge négative des éléments anioniques et opposée à elle.

9. Utilisation d'une nanoparticule zwitteronique selon l'une quelconque des revendications précédentes, **caractérisée en ce que** la nanoparticule est au moins entourée par une enveloppe.

10. Utilisation d'une nanoparticule zwitteronique selon l'une quelconque des revendications précédentes, **caractérisée en ce que** la liaison zwitteronique est décrite par la formule A-X-[L¹-Z¹-L²-Z²]ₙ avec
1. A étant au choix un groupe avec une affinité de surface de la nanoparticule ou un atome de valeur au moins égale à deux adapté pour former une liaison covalente avec la nanoparticule ;
2. L¹ et L² étant des groupes de lieurs indépendants l'un de l'autre ;
3. X représentant au choix un élément de jonction optionnel, X comportant en sus de la liaison en A au moins une autre liaison L¹;
4. n comprenant les nombres entiers entre 1 et la valence maximale de X-1 ;
5. Z¹ comprenant un premier groupe chargé ou ionisable ; et
6. Z² comprenant un deuxième groupe chargé ou ionisable pour lequel les charges Z¹ ou Z² sont opposées.

11. Utilisation d'une nanoparticule zwitteronique selon la revendication 10, **caractérisée en ce que** A est choisi dans le groupe C, N, O, S, P ou Si et prévoit au moins un groupe amino-, mercapto-, dithiocabamat-, carboxy, phosphat-, phosphonat-

12. Utilisation d'une nanoparticule zwitteronique selon la revendication 11, **caractérisée en ce que** L¹ et L² contiennent des atomes indépendants l'un de l'autre linéaires ou ramifiés, en option des chaînes d'hydrocarbures saturés ou insaturés avec un à quarante atomes C contenant au choix des hétéroatomes du groupe N, O, P, Si et S.

13. Utilisation d'une composition contenant au moins une nanoparticule zwitteronique selon l'une quelconque des revendications précédentes dans une solution aqueuse.

14. Utilisation selon l'une quelconque des revendications précédentes, l'application immunologique étant ou comprenant une modulation d'un allumage, la modulation d'un allumage étant ou comprenant notamment une réduction d'allumages auto-aggressifs.

15. Utilisation selon l'une quelconque des revendications précédentes, l'application immunologique étant ou comprenant une modulation d'une maladie auto-immune.

16. Utilisation selon l'une quelconque des revendications précédentes, l'application immunologique étant ou comprenant une modulation d'une allergie.

17. Utilisation selon l'une quelconque des revendications précédentes, l'application immunologique étant ou comprenant un sous-dosage de réponses immunologiques contre des éléments biologiques.

18. Utilisation selon l'une quelconque des revendications précédentes, l'application biologique étant une modulation d'une différenciation d'une cellule, notamment d'une cellule hépatique, par exemple d'une cellule endothéliale hépatique.
